(19) Europäisches Patentamt
European Patent Office
Office européen des brevets

(11) EP 1 077 947 B1

(12) EUROPÄISCHE PATENTSCHRIFT

(45) Veröffentlichungstag und Bekanntmachung des Hinweises auf die Patenterteilung:
**03.09.2003 Patentblatt 2003/36**

(21) Anmeldenummer: **99927741.1**

(22) Anmeldetag: **01.04.1999**

(51) Int Cl.^7: **C07D 231/56**, C07D 413/12, C07D 403/12, C07D 405/12, C07D 417/12, A61K 31/415, A61K 31/42, A61K 31/44, A61K 31/505

(86) Internationale Anmeldenummer:
**PCT/EP99/02289**

(87) Internationale Veröffentlichungsnummer:
**WO 99/058503 (18.11.1999 Gazette 1999/46)**

(54) **NEUE 1,5- UND 3-0-SUBSTITUIERTE 1H-INDAZOLE MIT ANTIASTHMATISCHER, ANTIALLERGISCHER, ENTZÜNDUNGSHEMMENDER, IMMUNMODULIERENDER UND NEUROPROTEKTIVER WIRKUNG, VERFAHREN ZU IHRER HERSTELLUNG UND IHRE VERWENDUNG ALS ARZNEIMITTEL**

NOVEL 1,5 AND 3-O-SUBSTITUTED 1H-INDAZOLES WITH ANTI-ASTHMATIC, ANTI-ALLERGIC, ANTI-INFLAMMATORY, IMMUNO-MODULATING AND NEURO-PROTECTIVE EFFECT, METHOD FOR THE PRODUCTION AND USE THEREOF AS MEDICAMENTS

NOUVEAUX 1H-INDAZOLES A SUBSTITUTION O AUX POSITIONS 1,5 ET 3, A EFFET ANTI-ASTHMATIQUE, ANTI-ALLERGIQUE, ANTI-INFLAMMATOIRE, IMMUNOMODULATEUR ET NEUROPROTECTEUR, LEUR PROCEDE DE PRODUCTION ET LEUR UTILISATION COMME MEDICAMENT

(84) Benannte Vertragsstaaten:
**AT BE CH CY DE DK ES FI FR GB GR IE IT LI LU MC NL PT SE**
Benannte Erstreckungsstaaten:
**LT LV MK RO SI**

(30) Priorität: **11.05.1998 DE 19821002**

(43) Veröffentlichungstag der Anmeldung:
**28.02.2001 Patentblatt 2001/09**

(73) Patentinhaber: **Arzneimittelwerk Dresden GmbH
01445 Radebeul (DE)**

(72) Erfinder:
- **SCHINDLER, Rudolf
D-01257 Dresden (DE)**
- **HÖFGEN, Norbert
D-01458 Medingen (DE)**
- **POPPE, Hildegard
D-01109 Dresden (DE)**
- **BRUNE, Kay
D-91080 Marloffstein (DE)**

(74) Vertreter: **Böhm, Brigitte, Dipl.-Chem. Dr. et al
Weickmann & Weickmann
Patentanwälte
Postfach 86 08 20
81635 München (DE)**

(56) Entgegenhaltungen:
**EP-A- 0 191 520**
**WO-A-96/04266**
**WO-A-97/34874**
**US-A- 3 470 194**
**US-A- 3 966 761**

Bemerkungen:
Die Akte enthält technische Angaben, die nach dem Eingang der Anmeldung eingereicht wurden und die nicht in dieser Patentschrift enthalten sind.

EP 1 077 947 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

### Technisches Gebiet

Die Erfindung betrifft die Herstellung und Verwendung von neuartigen Derivaten des Indazol-3-ols als Arzneimittel mit antiasthmatischen, antiallergischen, entzündungshemmenden, immunmodulierenden und neuroprotektiven Eigenschaften.

### Stand der Technik

Cyclosporin A (CsA) oder FK 506 sind immunsuppressive, von Pilzen stammende Naturstoffe, die den $Ca^{2+}$ -abhängigen Signalübertragungsweg in einigen Zelltypen inhibieren. In T-Zellen hemmen beide Verbindungen die Transkription einer Reihe von Genen. CsA und FK506 binden beide mit hoher Affinität an lösliche Rezeptorproteine wie zum Beispiel Cyclophilin (Cyp) oder FK-506 bindendes Protein (FKBP) (G. Fischer et al., Nature 337 (1989), 476-478; M. W. Harding et al., Nature 341 (1989), 755-760). Beide Proteine katalysieren die Isomerisierung von cis- und trans- Amidbindungsrotamasen von Peptiden und werden auch häufig als Immunophiline bezeichnet.
Der Komplex aus CsA-Cyp bzw. FK 506-FKBP bindet Calcineurin (CN) und inhibiert dessen Phosphataseaktivität. Als zelluläres Zielmolekül von CN wurde die cytosolische, phosphorylierende Komponente des Transkriptionsfaktors NF-AT erkannt, so daß bei fehlender CN-Aktivität der aktive Transkriptionskomplex am IL 2 Promoter nicht angeschaltet werden kann (M. K. Rosen, S. L. Schreiber, Angew. Chem. 104 (1992), 413-430, G. Fischer, Angew. Chem. 106 (1994), 1479-1501). Den allergischen, asthmatischen Erkrankungen liegt eine entzündliche Reaktion zugrunde, die von T-Zellen und ihren Mediatoren gesteuert wird. Corticosteroide stellen immer noch das Mittel der Wahl in der Behandlung vieler allergischer Erkrankungen dar. Auch CsA und FK 506 erwiesen sich sowohl im Tierexperiment als auch in klinischen Studien beim Asthma bronchiale und zugrunde liegenden Entzündungen als günstiges Therapeutikum.

Trotz der Vielzahl von Ansätzen zur Identifikation neuer aktiver Immunophilin-Inhibitoren konnten bisher keine wirksameren Strukturen als CsA, FK 506, Rapamycin bzw. Derivate von diesen Naturstoffen hergestellt bzw. isoliert werden. Das hohe inhibitorische Potential von CsA, FK 506 und Rapamycin wird jedoch ganz erheblich durch die mannigfaltigen Nebenwirkungen, insbesondere der Nieren- und Neurotoxizität, reduziert (N. H. Sigal et al., J. Exp. Med. 173 (1991), 619-6128). Hintergrund dieser Tatsache ist die Unspezifität der Wechselwirkung zwischen Immunophilin-Liganden und den zellspezifischen Bindungsproteinen. Dadurch ist die bekannte medizinisch-therapeutische Wirkung dieser Immunsuppressiva erheblich eingeschränkt. Ferner erweist sich die fehlende Selektivität der Verbindungen gerade in der Langzeittherapie als problematisch.

Substanzen, die die Aktivität von Peptidylprolylisomerasen (PPlasen) wie Cyp oder FKBP inhibieren, besitzen neuroprotektive Eigenschaften, stimulieren das neuronale Wachstum und sind zur Behandlung von neurodegenerativen Krankheiten geeignet (WO 96/40140, US 5,696,135, WO 97/18828).

Bekannt sind substituierte Indazol-Derivate, die sich jedoch hinsichtlich der Substituenten X, Y, Z, $R^1$, $R^2$ und $R^3$ und ihrer pharmakodynamischen Wirkung von den beanspruchten Verbindungen unterscheiden.

Corsi [Boll. Chim. Farm. 111, 566-57, 1972)] und Giannangeli et al. [Boll. Chim. Farm. 121, 465-474 (1982)] berichteten über die Synthese von 5-Hydroxy-Bendazac und EP-A-0 191 520 beschreibt die Verwendung der [(1-Benzyl-5-hydroxy-1Hindazol-3-yl)]-essigsäure zur Behandlung den Schnupfen.

Baiocchi et al. [Synthesis 1978 (9), 633-648] geben einen Überblick zu Synthesen und Eigenschaften der 1H-Indazol-3- ole.

WO97/34874 beschreibt 1,3,5-trisubstituierte Indazole mit antiasthmatischer, antiallergischer, entzündungshemmender und immunmodulierender Wirkung.

WO 96/04266 beinhaltet unter anderen mit basischen Resten substituierte (1H-Indazol-3-yloxy)acetamide und deren antiasthmatische, antiallergische, entzündungshemmende und immunmodulierende Eigenschaften.

Pfannstiel et al. [Ber. Dtsch. Chem. Ges. 75 (9), 1096-1107 (1942)] berichten über die Herstellung von Nitro-1H-indazol-3-olen.

Ketami et al. [J. of Heterocycl. Chem. 7 (4), 807-813 (1970)] beschreibt die antiinflammatorische Wirkung der 1,5-disubstituierten 3-Hydroxy-1H-indazole.

Hannig et al. [Pharmazie, 30 (11), 706-708] beschreiben 1-Benzylierte (Indazol-3-yl )-acylamino-carbonsäureanilide.

JP 48026760 beinhaltet unter anderem 1-Benzyl-1H-indazol-3-yloxyacetamide.

EP-A-0 290 145 umfaßt 1,3,6-trisubstituierte Indazole, die Leukotrien-Antagonisten darstellen.

US 3,470,194, Palazzo et al. [J. Med. Chem. 9, 38-41 (1966)] und Guyla et al [Acta pharm. Hung. 44, 49-57 (1974)] beschreiben 1,5-disubstituierte (Indazol-3-yl)-oxyalkansäuren und deren antiinflammatorische Aktivität.

Klicnar [Coll. Czech. Chem. Comm. 42, 327-337 (1977)] berichtet über physikalische Daten der Acyl-indazole.

Yamaguchi et al. [Chem. Pharm. Bull. 43 (2), 332-334 (1995)] beschreibt 3-O substituierte (1-Pyridin-3-yl)-indazole und deren antiasthmatische Wirkung.

EP 0 448 206 umfaßt 1,3,6-trisubstituierte Indazole und ihre Verwendung als Herbizide, wobei in 3-Position nur OH bzw. O-Alkyl zugelassen sind.

EP 0 191 520 beschreibt (1-Phenylmethyl)-5-hydroxy-1H-indazol-3-yl)-oxyessigsäure und dessen pharmazeutische Verwendung.

EP 0 199 543 beschreibt Indazole und andere Heterocyclen mit sauren Substituenten im Rest M als Leukotrien-Antagonisten.

US 3,966,761 beinhaltet trisubstituierte Amino-indazole und deren antiinflammatorische und analgetische Effekte.

US 3,318,905 beschreibt 3-Dialkylaminoalkyloxy-indazole mit analgetischer und antiphlogistischer Aktivität.

K. v. Auwers [Ber. Dtsch. Chem. Ges. 58, 2081-2088 (1925)] beschreibt die Konstitution von Acyl-indazolen.

Zoni et al. [Il Farmaco Ed. Sci. 23 (5), 490-501 (1968)] und Zoni et al. [Boll. Chim. Farm. 107, 598-605 (1968)] beschreiben die Alkylierung von 1-substituierten 1H-Indazol-3-olen.

Evans et al. [Tetrahedron 21, 3351-3361 (1965)] beschreiben die Synthese von 1,3-substituierten Acyl- und Tosyl-indazolen.

Anderson et al. [J. Chem. Soc. C , 3313-3314 (1971)] beschreiben 1,3-substituierte Tosyl-indazole.

Schmutz et al. [Helv. Chim. Acta 47, 1986-1996 (1964)] beschreibt die Alkylierung von Indazolonen.

Zur Behandlung von asthmatischen Erkrankungen besteht aufgrund zahlreicher Nebenwirkungen der eingeführten Präparate, mangelnder Heilerfolge und der bislang zu unspezifischen Therapie weiterhin ein großes Bedürfnis an Verbindungen mit einer hohen Effektivität und Sicherheit.

Der Erfindung liegt die Aufgabe zugrunde, neue Verbindungen mit Rotamase inhibierenden und/oder die pulmonalen Eosinophilen-Infiltration hemmenden Eigenschaften zu finden und durch gezielte Synthese bereitzustellen.

Eine völlig neuartige Substanzklasse, die Immunophiline überraschenderweise spezifisch bindet, wird durch die erfindungsgemäßen Verbindungen der Formel **I** dargestellt. Diese Klasse von Verbindungen weist eine hohe Affinität zu Immunophilinen wie CypB auf.

Darstellung der Erfindung

Überraschenderweise wurde nun gefunden, daß die neuen Indazol-Derivate in der Lage sind, die Wirkung der PPlase zu inhibieren. Demzufolge sind diese Verbindungen für die Herstellung von Arzneimitteln von großer Bedeutung, wo die Hemmung der PPlase von Nutzen ist. Solche Krankheiten sind zum Beispiel: periphere Neuropathien, Neurodegeneration, Schlaganfall, Parkinson und Alzheimer Erkrankungen, traumatische Gehirnerkrankungen, Multiple Sclerose.

Weiterhin wurde nachgewiesen, daß die erfindungsgemäßen Verbindungen in der Lage sind, die für die asthmatische late phase Reaktion charakteristische Einwanderung von eosinophilen Granulozyten in das Gewebe zu inhibieren.

Die Erfindung betrifft neue 1,5- und 3-O- substituierte 1H-Indazole der allgemeinen Formel **I**

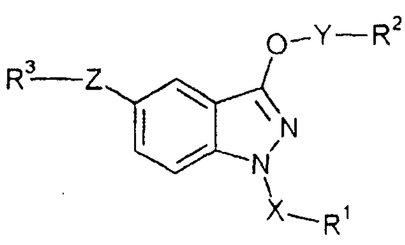

**Formel I**

worin X, Y, Z ,$R^1$, $R^2$ und $R^3$ folgende Bedeutung haben:

X kann $-SO_2-$, $-(CH_2)_p-$, $-(CH_2)_p-O-$ mit p = 1...4 sein,

Y kann $-(CH_2)_p-$, $-(CH_2)_p-O-$, $-(CH_2)_p-(C{=}O)-$, $-(CH_2)_p-(C{=}O)-NH-$, $-(CH_2)_p-CHOH-$, $-(CH_2)_p-CH{=}CH-$, mit p = 1...4 sein,

Z kann -O-, -S-, -NH-(C=O)-NH- sein,

$R^1$ kann folgende Bedeutung haben:
Naphthyl, Chinolyl oder Phenyl, wobei die Carbocyclen substituiert sein können mit:
$-C_{1...6}$-Alkyl, $-O-C_{1...6}$-Alkyl, -F, -Cl, $-NO_2$, $-NH_2$, $-(CO)C_{1...6}$-Alkyl, $-S-C_{6...14}$-Aryl,

$R^1$ kann weiter H sein, mit der Maßgabe, dass $R^1$ nicht H ist, wenn X = $CH_2$;

$R^2$ kann folgende Bedeutung haben:
Phenyl, oder mono- oder bicyclische gesättigte oder ein- oder mehrfach ungesättigte Heterocyclen mit 5...15 Ringgliedern und 1...6 Heteroatomen; die vorzugsweise N, O und S sind, insbesondere Thiophenyl, Pyridinyl, isoxazolyl, Benzimidazolyl, Benz[1,3]dioxolyl, Pyrimidinyl, Chinolyl, Chinazolinyl, Morpholinyl, Pyrrolidinyl, Pyrrolyl, Phenyl[1,2,4]oxadiazolyl, Phenylthiazolyl,
wobei das Phenyl bzw. die Heterocyclen einfach oder mehrfach substituiert sein können mit:
$-C_{1...6}$-Alkyl, $-O-C_{1...6}$-Alkyl, monocyclische gesättigte Carbocyclen mit 3...14 Ringgliedern, -F, -Cl, -Br, $-NO_2$, $-NH_2$, -CN, $-(CO)C_{1...6}$-Alkyl, Benzyloxy, $-CF_3$,

$R^3$ kann folgende Bedeutung haben:
Methyl, Napthyl oder Phenyl, wobei das Phenyl einfach oder mehrfach substituiert sein kann mit:
$-O-C_{1...6}$-Alkyl, -Cl.

Die erfindungsgemäßen Verbindungen sind neu, jedoch ausgenommen Verbindungen gemäß Formel I:
Wenn Y = $-(CH_2)_p-(C=O)-$, $-(CH_2)_p-(C=O)-NH-$ mit p = 1...4, dann darf $R^2$ nicht gleich Pyridin, Piperazin, Pyrimidin, Tetrahydropyridin sein;

Weiterhin betrifft die Erfindung

1,5- und 3-O-substituierte 1H-Indazole, ausgewählt aus 3-[2-(2-Brom-4,6-difluorphenoxy)ethoxy]- 5-methoxy-1-(toluol-4-sulfonyl)-1H-indazol, 5-Methoxy-3-[2-(phenoxy)ethoxy]-1-(toluol-4-sulfonyl)-1H-indazol, 5-Methoxy-3-[3-(phenoxy)propoxy]-1-(toluol-4-sulfonyl)-1H-indazol3-[4-(Butoxy)propoxy]-5-methoxy-1-(toluol-4-sulfonyl)-1H-indazol, 3-[2-(2-Bromphenoxy)ethoxy]-5-methoxy-1-(toluol-4-sulfonyl)-1H-indazol, 3-[2-(4-Chlorphenoxy)ethoxy]-5-methoxy-1-(toluol-4-sulfonyl)-1H-indazol, 3-[2-(4-Chlorphenoxy)ethoxy]-5-methoxy-1-(4-chlorbenzensulfonyl)-1Hindazol, 3-[2-(4-Chlorphenoxy)ethoxy]-5-methoxy-1-(4-fluorbenzensulfonyl)-1H-indazol, 3-[2-(4-Chlorphenoxy)ethoxy]-5-methoxy-1-(4-methoxybenzensulfonyl)-1H-indazol, 3-[2-(4-Chlorphenoxy)ethoxy]-5-methoxy-1-(4-trifluormethoxybenzensulfonyl)-1H-indazol, 3-[2-(2,6-Dichlorphenoxy)ethoxy]-5-methoxy-1-(toluol-4-sulfonyl)-1H-indazol, 3-[2-(2,4-Difluorphenoxy)ethoxy]-5-methoxy-1-(toluol-4-sulfonyl)-1H-indazol, 3-[2-(2,6-Difluorphenoxy)ethoxy]-5-methoxy-1-(toluol-4-sulfonyl)-1 H-indazol, 5-Methoxy-1-(toluol-4-sulfonyl)-3-[2-(2,4,6-trifluorphenoxy)ethoxy]-1H-indazol,3-[2-(2-Brom-4,6-Difluorphenoxy)ethoxy]-5-methoxy-1-(4-methoxybenzensulfonyl)-1H-indazol,3-[2-(2-Brom-4,6-Difluorphenoxy)ethoxy]-5-methoxy-1-(4-chlorbenzensulfonyl)-1H-indazol, 3-[2-(2,6-Dibrom-4-nitrophenoxy)ethoxy]-5-methoxy-1-(toluol-4-sulfonyl)-1H-indazol,3-[2-(2,6-Dibrom-4-nitrophenoxy)ethoxy]-5-methoxy-1-(4-methoxybenzensulfonyl)-1H-indazol,5-Methoxy-3-[2-(4-nitrophenoxy)ethoxy]-1-(toluol-4-sulfonyl)-1H-indazol,1-(4-Chlorbenzensulfonyl)-5-methoxy-3-[2-(4-nitrophenoxy)ethoxy]- 1H-indazol, 5-Methoxy-1-(4-methoxybenzensulfonyl)-3-[2-(4-nitrophenoxy)ethoxy]-1H-indazol, 5-Methoxy-3-[2-(4-nitrophenoxy)ethoxy]-1-(thiophen-2-sulfonyl)-1H-indazol, 3-[2-(4-Cyanophenoxy)ethoxy]-5-methoxy-1-(toluol-4-sulfonyl)-1H-indazol, 3-[2-(4-Carboxamidophenoxy)ethoxy]-5-methoxy-1-(toluol-4-sulfonyl)-1Hindazol, 5-Methoxy-1-(toluol-4-sulfonyl)-3-[5-(3-trifluormethylphenyl)-1,2,4-oxadiazol-3-ylmethoxy]-1H-indazol, 3-(4-Benzyloxy-benzyloxy)-5-methoxy-1-(toluol-4-sulfonyl)-1H-indazol, 3-(5-Acetyl-2-methoxybenzyloxy)-5-methoxy-1-(toluol-4-sulfonyl)-1H-indazol, 3-(2-Chlor-6-fluorbenzyloxy)-5-methoxy-1-(toluol-4-sulfonyl)-1H-indazol, 1-(4-Chlorbenzensulfonyl)- 3-(2-chlor-6-fluorbenzyloxy)-5-methoxy-1H-indazol, 1-(4-Fluorbenzensulfonyl)-5-methoxy-3-(3-trifluormethylbenzyloxy)-1H-indazol, 3-(2-Fluorbenzyloxy)-5-methoxy-1-(toluol-4-sulfonyl)-1H-indazol,1-(4-Fluorbenzensulfonyl)-3-(2-fluorbenzyloxy)-5-methoxy-1H-indazol,1-(4-Fluorbenzensulfonyl)-3-(4-fluorbenzyloxy)-5-methoxy-1H-indazol,3-(4-Chlor-2-nitrobenzyloxy)-5-methoxy-1-(toluol-4-sulfonyl)-1H-indazol, 3-(6-Chlor-benzo[1,3]dioxo-5-ylmethoxy)-5-methoxy-1-(toluol-4-sulfonyl)-1H-indazol, 3-(6-Fluor-4Hbenzo[1,3]dioxin-8-ylmethoxy)-5-methoxy-(toluol-4-sulfonyl)-1Hindazol, 5-Methoxy-3-(6-nitro-4H-benzo[1,3]dioxin-8-ylmethoxy)-1-(toluol-4-sulfonyl)-1H-indazol, 5-Methoxy-3-(6-nitro-4Hbenzo[1,3]dioxin-8-ylmethoxy)-1-(4-fluorbenzensulfonyl)-1H-indazol3-(3,5-Dimethyl-isoxazol-4-ylmethoxy)-5-methoxy-1-(toluol-4-sulfonyl)-1H-indazol, 3-(3,5-Dimethyl-isoxazol-4-ylmethoxy)-5-methoxy-1-(4-chlorbenzensulfonyl)-1H-indazol, 5-Methoxy-3-[5-(2-methoxyphenyl)[1,2,4]oxadiazol-3-ylmethyl]-1-(toluol-4-sulfonyl)-1Hindazol, 5-Methoxy-3-[5-(4-tert.-butylphenyl)[1,2,4]oxadiazol-3-ylmethyl]-1-(toluol-4-sulfonyl)-1H-indazol, 3-[2-(4-Chlorphenyl)-thiazol-4-ylmethyl]- 5-methoxy-1-(toluol-4-sulfonyl)-1H-indazol, 6-{2-[5-Methoxy-1-(toluol-4-sulfonyl)-1H-indazol-3-yloxy]acetyl}-3,4-dihydro-1H-chinolin-2-on, 3-[2-(4-Chlorphenyl)-2-oxo-ethoxy]-5-methoxy-1-(toluol-4-sulfonyl)-1H-indazol, 3-[2-(4-Chlorphenyl)-2-oxo-ethoxy]-5-methoxy-1-(4-methoxybenzensulfonyl)-1H-indazol, 3-[2-(4-Chlorphenyl)-2-oxo-ethoxy]-5-methoxy-1-(4-chlorbenzenesulfonyl)-1H-indazol, 3-[2-(3,4-Dichlorphenyl)-2-oxo-ethoxy]-5-methoxy-1-(toluol-4-sulfonyl)-1Hindazol, 3-[2-(3,4-Dichlorphenyl)-2-oxo-ethoxy]-5-methoxy-1-(4-chlorbenzensulfonyl)-1H-indazol, 3-[2-(3,4-Dichlorphenyl)-2-oxoethoxyl-5-methoxy-1-(4-acetylamino-benzensulfonyl)-1H-indazol,3-[2-(3,4-Dichlorphenyl)-2-oxo-ethoxy]-5-methoxy-1-(naphthalen-1-sulfonyl)-1H-indazol, 3-[2-(3,4-Dichlorphenyl)-2-oxo-ethoxy]-5-methoxy-1-(chinolin-8-sulfonyl)-1H-indazol, 3-[2-(4-Diphenyl)-2-oxo-ethoxy]-5-methoxy-1-(toluol-4-sulfonyl)-1H-indazol, 3-[2-(4-Diphenyl)-2-oxoethoxy]-5-methoxy-1-(4-chlorbenzensulfonyl)-1H-indazol, 3-[2-(4-Diphenyl)-2-oxo-ethoxy]-5-methoxy-1-(4-methoxybenzensulfonyl)-1Hindazol, 3-[2-(4-Diphenyl)-2-oxo-ethoxy]-5-methoxy-1-(4-acetylaminobenzensulfonyl)-1H-indazol, N-[2,4-Difluorphenyl)-2-[5-methoxy-1-(toluol-4-sulfonyl)-1H-indazol-3-yloxy]-acetamid, 5-Methoxy-3-(3-phenylallyloxy)-1-(toluol-4-sulfonyl)-1H-indazol, 1-(2,4-Dichlorphenyl)-2-[5-methoxy-1-(toluol-4-sulfonyl)-1H-indazol-3-yloxy]-ethanol, 1-(4-Methoxyphenyl)-4-[5-methoxy-1-(toluol-4-sulfonyl)-1H-indazol-3-yloxy]-butan-1-on, 1-(4-Methoxyphenyl)-4-[1-(4-fluorbenzensulfonyl)-5-methoxy-1H-indazol-3-yloxy]-butan-1-on, 1-(4-Fluorphenyl)-4-[5-methoxy-1-(toluol-4-sulfonyl)-1H-indazol-3-yloxy]-butan-1-on, N-(4-Methoxyphenyl)-2-[5-methoxy-1-(toluol-4-sulfonyl)-1H-indazol-3-yloxy]-acetamid,N-(2,4-Dichlorphenyl)-2-[5-methoxy-1-(toluol-4-sulfonyl)-1Hindazol-3-yloxy]-acetamid,N-Benzyl-2-[5-methoxy-1-(toluol-4-sulfonyl)-1H-indazol-3-yloxy]-acetamid, 3-{2-[5-Methoxy-1-(toluol-4-sulfonyl)-1Hindazol-3-yloxy]ethyl}-1H-chinazolin-2,4-dion, 3-{2-[5-Methoxy-1-(4-methoxybenzensulfonyl)-1H-indazol-3-yloxy]ethyl}-1H-chinazolin-2,4-dion, 3-(6-Chlorbenzo [1,3] dioxol-5-ylmethoxy)-5-methoxy-1-(3-nitrobenzyl)-1H-indazol,3-[2-(4-Chlorphenoxy)ethoxy]-5-methoxy-1-(3-nitrobenzyl)-1H-indazol,3-[2-(4-Nitrophenoxy)ethoxy]-5-methoxy-1-(3-nitrobenzyl)-1H-indazol, 3-[2-(4-Carboxaminophenoxy) ethoxy]-5-methoxy-1-(3-nitrobenzyl)-1H-indazol,3-[2-(2-Brom-4,6-difluorphenoxy)ethoxy]-5-methoxy-1-(3-nitrobenzyl)-1H-indazol, 3-[2-(2,6-Dibrom-4-nitrophenoxy)ethoxy]-5-methoxy-1-(3-nitrobenzyl)-1Hindazol, 5-Methoxy-1-(3-nitrobenzyl)-3-(pyridin-4-ylmethoxy)-1H-indazol Hydrochlorid, 3-(2,6-Dichlorbenzyloxy)-5-methoxy-1-(3-nitrobenzyl)-1Hindazol,3-[2-(4-Chlorphenyl)acetoxy]-5-methoxy-1-(3-nitrobenzyl)-1Hindazol, 3-[3-(6-Chlorbenzo [1,3] dioxol-5-ylmethoxy)-5-methoxyindazol-1-ylmethyl]-phenylamin, 4-{2-[1-(3-Aminobenzyl)-5-methoxy-1H-indazol-3-yloxy]-ethoxy}-phenylamin Dihydrochlorid, 4-{2-[1-(3-Aminobenzyl)-5-methoxy-1H-indazol-3-yloxy]-ethoxy}-benzamid, 3-[3-(4-Chlorphenylcarboxymethyloxy)-5-methoxy-indazol-1-ylmethyl]-phenylamin Hydrochlorid, 1-(4-Fluorbenzyl)-5-methoxy-3-[2-(4-nitrophenoxy)ethoxy]-1H-indazol, 3-[2-(4-Chlorphenoxy)ethoxy]-1-(4-fluorbenzyl)-5-methoxy-1H-indazol, 3-[2-(4-Aminophenoxy)ethoxy]-1-(4-fluorbenzyl)-5-methoxy-1H-indazol, 3-[2-(2-Brom-4,6-difluorphenoxy) ethoxy]-1-(4-fluorbenzyl)-5-methoxy-1H-indazol, 3-(3,5-Dimethylisoxazol-4-ylmethoxy)-1-(4-fluorbenzyl)-5-methoxy-1H-indazol, 3-(2,3,6-Trifluorbenzyloxy)-1-(4-fluorbenzyl)-5-methoxy-1H-indazol,3-(6-Chlorbenzo[1,3]dioxol-5-ylmethoxy)-1-(4-fluorbenzyl)-5-methoxy-1Hindazol, 3-(4-Chlorphenylcarboxymethyloxy)-1-(4-fluorbenzyl)-5-methoxy-1H-indazol,3-[2-(2-Brom-4,6-difluorphenoxy)ethoxy]-5-methoxy-1-[2-(4-nitrophenoxy)ethyl]-1H-indazol,3-[2-(4-Chlorphenoxy)ethoxy]-5-methoxy-1-[2-(4-nitrophenoxy)ethyl]-1Hindazol, 3-[2-(2,6-Dibrom-4-nitrophenoxy)ethoxy]-5-methoxy-1-[2-(4-nitrophenoxy)ethyl]-1H-indazol,3-(4-Chlorphenylcarboxymethyloxy)-5-methoxy-1-[2-(4-nitrophenoxylethyl]-1H-indazol,3-(3,4-Dichlorphenylcarboxymethyloxy)-5-methoxy-1-[2-(4-nitrophenoxy)ethyl]-1H-indazol, 3-(4-Diphenylcarboxymethyloxy)-5-methoxy-1-[2-(4-nitrophenoxy)ethyl]-1H-indazol, 6-(2-{5-Methoxy-1-[2-(4-nitrophenoxy)ethyl]-1H-indazol-3-yloxy}acetyl]-3,4-dihydro-1Hchinolin-2-on, N-(2,4-Difluorphenyl)-2-[5-methoxy-1-[2-(4-nitrophenoxy) ethyl]-1H-indazol-3-yloxy}acetamid, 3-(3-Acetyl-6-methoxybenzyloxy)-5-methoxy-1-[2-(4-nitrophenoxy)ethyl]-1H-indazol, 3-(3-Trifluormethylbenzyloxy)-5-methoxy-1-[2-(4-nitrophenoxy)ethyl]-1H-indazol, 3-(2-Fluormethylbenzyloxy)-5-methoxy-1-[2-(4-nitrophenoxy)ethyl]-1H-indazol,3-(6-Fluor-4H-benzo[1,3]dioxin-8-ylmethoxy)-5-methoxy-1-[2-(4-nitrophenoxy)ethyl]-1H-indazol,3-[2-(4-Chlorphenoxy)ethoxy]- 1-[2-(4-chlorphenoxy)ethyl]-5-methoxy-1Hindazol, 3-(4-Chlorphenoxyalkyloxy)-5-amino-1-(toluol-4-sulfonyl)-1Hindazol, 3-(2,6-Dibrom-4-nitrophenoxyalkyloxy)-5-amino-1-(toluol-4-sulfonyl)-1H-indazol und 3-(4-Chlorphenylcarboxymethyloxy)-5-amino-1-(toluol-4-sulfonyl)-1H-indazol.

Bevorzugt sind die Verbindungen

3-[2-(2-Brom-4,6-difluorphenoxy)ethoxy]-5-methoxy-1-(toluol-4-sulfonyl)-1H-indazol,
5-Methoxy-1-(toluol-4-sulfonyl)-3-[5-(3-trifluormethylphenyl)-1,2,4-oxadiazol-3-ylmethoxy]-1H-indazol,
6-{2-[5-Methoxy-1-(toluol-4-sulfonyl)-1H-indazol-3-yloxy]acetyl}-3,4-dihydro-1 H-chinolin-2-on,
N-(2,4-Difluorphenyl)-2-[5-methoxy-1-(toluol-4-sulfonyl)-1H-indazol-3-yloxy]-acetamid,

3-(6 Chlorbenzo[1,3]dioxol-5-ylmethoxy)-5-methoxy-1-(3-nitrobenzyl) -1H-indazol,
3-[3-(4-Fluorphenyl)-propoxy]-5-nitro-1-(3-nitrobenzyl)-1H-indazol,
3-[3-(6-Chlorbenzo [1,3] dioxol-5-ylmethoxy)-5-methoxy-indazol -1-ylmethyl]-phenylamin,
1-(4-Fluorbenzyl)-5-methoxy-3-[2-(4-nitrophenoxy)ethoxy]-1H-indazol,
3-[2-(2-Brom-4,6-difluorphenoxy)ethoxy]-5-methoxy-1-[2-(4-nitrophenoxy)ethyl]-1H-indazol,
3-[2-(2-Brom-4,6-difluorphenoxy)ethoxy]-1-[3,4-dichlorbenzyl]-5-methylthio-1H-indazol und
1-1-{1-(2,4-Dichlorbenzyl)-3-[2-(4-nitrophenoxy)ethoxyl-1H -indazol-5-yl}-3-naphthalen-1-ylharnstoff.

Besonders bevorzugt sind die Verbindungen

3-[2-(2-Brom-4,6-difluorphenoxy)ethoxy]5-methoxy-1-(toluol-4-sulfonyl)-1 H-indazol und
3-[2-(4-Chlorphenoxy)ethoxy]-5-methoxy-1-1(4-fluorbenzensulfonyl)-1H-indazol ist.

Weiterhin betrifft die Erfindung die physiologisch verträglichen Salze der Verbindungen gemäß Formel I. Die pharmakologisch verträglichen Salze werden in üblicher Weise durch Neutralisation der Basen mit anorganischen oder organischen Säuren bzw. durch Neutralisation der Säuren mit anorganischen oder organischen Basen erhalten. Als anorganische Säuren kommen zum Beispiel Salzsäure, Schwefelsäure, Phosphorsäure oder Bromwasserstoffsäure, als organische Säuren zum Beispiel Carbon-, Sulfo- oder Sulfonsäure wie Essigsäure, Weinsäure, Milchsäure, Propionsäure, Glykolsäure, Malonsäure, Maleinsäure, Fumarsäure, Gerbsäure, Succinsäure, Alginsäure, Benzoesäure, 2-Phenoxybenzoesäure, 2-Acetoxybenzosäure, Zimtsäure, Mandelsäure, Zitronensäure, Apfelsäure, Salicylsäure, 3-Aminosalicylsäure, Ascorbinsäure, Embonsäure, Nicotinsäure, Isonicotinsäure, Oxalsäure, Aminosäuren, Methansulfonsäure, Ethansulfonsäure, 2-Hydroxyethansulfonsäure, Ethan-1,2-disulfonsäure, Benzolsulfonsäure, 4-Methylbenzolsulfonsäure oder Naphthalin-2-sulfonsäure in Frage. Als anorganische Basen kommen zum Beispiel Natronlauge, Kalilauge, Ammoniak sowie als organische Basen Amine, bevorzugt jedoch tertiäre Amine, wie Trimethylamin, Triethylamin, Pyridin, N,N-Dimethylanilin, Chinolin, Isochinolin, $\alpha$-Picolin, $\beta$-Picolin, $\gamma$-Picolin, Chinaldin oder Pyrimidin in Frage.
Desweiteren können physiologisch verträgliche Salze der Verbindungen gemäß Formel **I** dadurch gewonnen werden, daß Derivate, die tertiäre Amino-Gruppen besitzen, in an sich bekannter Weise mit Quaternierungsmitteln in die entsprechenden quaternären Ammoniumsalze überführt werden. Als Quaternierungsmittel kommen beispielsweise Alkylhalogenide wie Methyliodid, Ethylbromid und n-Propylchlorid, aber auch Arylalkylhalogenide wie Benzylchlorid oder 2-Phenylethylbromid in Frage.
Weiterhin betrifft die Erfindung von Verbindungen der Formel **I** , die ein asymmetrisches Kohlenstoffatom enthalten , die D-Form, die L-Form und
D,L-Mischungen sowie im Falle mehrerer asymmetrischer Kohlenstoffatome die diastereomeren Formen. Diejenigen Verbindungen der Formel **I**, die asymmetrische Kohlenstoffatome enthalten und in der Regel als Razemate anfallen , können in an sich bekannter Weise beispielsweise mit einer optisch aktiven Säure in die optisch aktiven Isomeren getrennt werden . Es ist aber auch möglich , von vornherein eine optisch aktive Ausgangssubstanz einzusetzen , wobei dann als Endprodukt eine entsprechende optisch aktive beziehungsweise diastereomere Verbindung erhalten wird.
Gegenstand der Erfindung ist die Verwendung der erfindungsgemäßen Verbindungen bzw. deren physiologisch verträglichen Salze als

1. Inhibitoren der PPlase zur Herstellung von Arzneimitteln zur Behandlung von durch dieses Enzym vermittelten Erkrankungen und / oder
2. Inhibitoren der Spätphasen-Eosinophilie zur Herstellung von Arzneimitteln zur Behandlung von durch diese Zellen vermittelten Erkrankungen.

Zu diesen Erkrankungen gehören beispielsweise Asthma bronchiale, allergische Rhinitis, allergische Konjunktivitis, atopische Dermatitis, Ekzeme, allergische Angiitis, durch Eosinophile vermittelte Entzündungen wie eosinophile Fasciitis, eosinophile Pneumonie und PIE-Syndrom, Autoimmunerkrankungen wie rheumatoide Arthritis, rheumatoide Spondylitis, Lupus erythematosus, Multiple Sclerose, Psoriasis, Glomerulonephritis und Uveitis, Insulin-abhängiger Diabetes mellitus und Sepsis.
Die erfindungsgemäßen Verbindungen bzw. deren physiologisch verträgliche Salze werden weiterhin zur Herstellung von Arzneimitteln zur Verhinderung von Abstoßungsreaktionen nach Transplantationen von Zellen, Geweben oder Organen verwendet.
Zur Herstellung der Arzneimittel wird neben den üblichen Hilfsmitteln, Träger- und Zusatzstoffen eine wirksame Dosis der erfindungsgemäßen Verbindungen oder deren Salze verwendet.
Die Dosierung der Wirkstoffe kann je nach Verabfolgungsweg, Alter, Gewicht des Patienten, Art und Schwere der zu behandelnden Erkrankungen und ähnlichen Faktoren variieren.
Die tägliche Dosis kann als einmal zu verabreichende Einzeldosis oder unterteilt in 2 oder mehrere Tagesdosen gegeben werden und beträgt in der Regel 0,001-1000 mg.
Als Applikationsform kommen orale, parenterale, intravenöse, transdermale, topische, inhalative und intranasale Zubereitungen in Frage.
Zur Anwendung kommen die üblichen galenischen Zubereitungsformen wie Tabletten, Dragees, Kapseln, dispergierbare Pulver, Granulate, wäßrige Lösungen, Aerosole , Pulverformulierungen, Pflaster, Lösungen, Ampullen, Suppositorien, wässrige oder ölige Suspensionen, Sirup, Säfte oder Tropfen.
Feste Arzneiformen können inerte Inhalts- und Trägerstoffe enthalten, wie z. B. Calciumcarbonat, Calciumphosphat, Natriumphosphat, Lactose, Stärke, Mannit, Alginate, Gelatine, Guar-gummi, Magnesium- oder Aluminiumstearat, Methylcellulose, Talkum, hochdisperse Kieselsäuren, Silikonöl, höhermolekulare Fettsäuren (wie Stearinsäure), Gelatine, Agar-Agar oder pflanzliche oder tierische Fette und Öle, feste hochmolekulare Polymere (wie Polyethylenglykol); für orale Applikation geeignete Zubereitungen können gewünschtenfalls zusätzliche Geschmacks- und/oder Süßstoffe enthalten.

Flüssige Arzneiformen können sterilisiert sein und/oder gegebenenfalls Hilfsstoffe wie Konservierungsmittel, Stabilisatoren, Netzmittel, Penetrationsmittel, Emulgatoren, Spreitmittel, Lösungsvermittler, Salze, Zucker oder Zuckeralkohole zur Regelung des osmotischen Drucks oder zur Pufferung und/oder Viskositätsregulatoren enthalten. Derartige Zusätze sind zum Beispiel Tartrat- und Citrat-Puffer, Ethanol, Komplexbildner (wie Ethylendiamin-tetraessigsäure und deren nicht-toxische Salze). Zur Regelung der Viskosität kommen hochmolekulare Polymere in Frage wie beispielsweise flüssiges Polyethylenoxid, mikrokristalline Cellulosen Carboxymethylcellulosen, Polyvinylpyrrolidone, Dextrane oder Gelatine. Feste Trägerstoffe sind zum Beispiel Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere wie Polyethylenglykol.

Ölige Suspensionen für parenterale oder topische Anwendungen können vegetabile synthetische oder semisynthetische Öle wie beispielsweise flüssige Fettsäureester mit jeweils 8 bis 22 C-Atomen in den Fettsäureketten, zum Beispiel Palmitin-, Laurin-, Tridecyl-, Margarin-, Stearin-, Arachin-, Myristin-, Behen-, Pentadecyl-, Linol-, Elaidin-, Brasidin-, Eruca- oder Ölsäure, die mit ein- bis dreiwertigen Alkoholen mit 1 bis 6 C-Atomen wie beispielsweise Methanol, Ethanol, Propanol, Butanol, Pentanol oder deren Isomere, Glycol oder Glycerol verestert sind, sein. Derartige Fettsäureester sind beispielsweise handelsübliche Miglyole, Isopropylmyristat, Isopropylpalmitat, Isopropylstearat, PEG 6-Caprinsäure, Capryl/Caprinsäureester von gesättigten Fettalkoholen, Polyoxyethylenglyceroltrioleate, Ethyloleat, wachsartige Fettsäureester wie künstliches Entenbürzeldrüsenfett, Kokosfettsäure-isopropylester, Ölsäureoleylester, Ölsäuredecylester, Milchsäureethylester, Dibutylphthalat, Adipinsäurediisopropylester, Polyol-Fettsäureester u. a. Ebenso geeignet sind Silikonöle verschiedener Viskosität oder Fettalkohole wie Isotridexylalkohol, 2-Octyldodecanol, Cetylstearyl-Alkohol oder Oleylalkohol, Fettsäuren wie beispielsweise Ölsäure. Weiterhin können vegetabile Öle wie Rizinusöl, Mandelöl, Olivenöl, Sesamöl, Baumwollsaatöl, Erdnußöl oder Sojabohnenöl Verwendung finden.

Als Lösungsmittel, Gelbildner und Lösungsvermittler kommen in Frage Wasser oder mit Wasser mischbare Lösungsmittel. Geeignet sind zum Beispiel Alkohole wie beispielsweise Ethanol oder Isopropylalkohol, Benzylalkohol, 2-Octyldodecanol, Polyethylenglykole, Phthalate, Adipate, Propylenglykol, Glycerin, Di- oder Tripropylenglykol, Wachse, Methylcellosolve, Cellosolve, Ester, Morpholine, Dioxan, Dimethylsulfoxid, Dimethylformamid, Tetrahydrofuren, Cyclohexanon etc.

Als Filmbildner können Celluloseether verwendet werden, die sich sowohl in Wasser als auch in organischen Lösungsmitteln lösen bzw. anquellen können, wie beispielsweise Hydroxypropylmethylcellulose, Methylcellulose, Ethylcellulose oder lösliche Stärken.

Mischformen zwischen Gel- und Filmbildnern sind durchaus ebenfalls möglich. Hier kommen vor allem ionische Makromoleküle zur Anwendung, wie z. B. Natriumcarboxymethylcellulose, Polyacrylsäure, Polymethacrylsäure und deren Salze, Natriumamylopektinsemiglykolat, Alginsäure oder Propylenglykol-Alginat als Natriumsalz, Gummi arabicum, Xanthan-Gummi, Guar-Gummi oder Carrageenan.

Als weitere Formulierungshilfsmittel können eingesetzt werden: Glycerin, Paraffin unterschiedlicher Viskosität, Triethanolamin, Collagen, Allantoin, Novantisolsäure. Auch die Verwendung von Tensiden, Emulgatoren oder Netzmitteln kann zur Formulierung notwendig sein, wie z. B. von Na-Laurylsulfat, Fettalkoholethersulfaten, Di-Na-N-lauryl-ß-iminodipropionat, polyoxyethyliertes Rizinusöl oder Sorbitan-Monooleat, Sorbitan-Monostearat, Polysorbaten (z. B. Tween), Cetylalkohol, Lecithin, Glycerinmonostearat, Polyoxyethylenstearat, Alkylphenolpolyglykolether, Cetyltrimethylammoniumchlorid oder Mono-/Dialkylpolyglykolether-orthophosphorsäure-monoethanolaminsalzen. Stabilisatoren wie Montmorillonite oder kolloidale Kieselsäuren zur Stabilisierung von Emulsionen oder zur Verhinderung des Abbaus der aktiven Substanzen wie Antioxidantien, beispielsweise Tocopherole oder Butylhydroxyanisol, oder Konservierungsmittel, wie p-Hydroxybenzoesäureester, können ebenfalls zur Zubereitung der gewünschten Formulierungen gegebenenfalls erforderlich sein.

Die Herstellung, Abfüllung und Verschließung der Präparate erfolgt unter den üblichen antimikrobiellen und aseptischen Bedingungen.

Die Dosierung der pharmazeutischen Zubereitungen hängt vom Alter, Zustand und Gewicht des Patienten sowie von der Applikationsform ab. In der Regel beträgt die tägliche Wirkstoffdosis zwischen 0,001-25 mg/kg Körpergewicht.

<u>Herstellung</u>

Entsprechend der vorliegenden Erfindung können die Verbindungen der allgemeinen Formel I nach folgenden Verfahren hergestellt werden:

Verfahren zur Herstellung der Verbindungen der allgemeinen Formel I, gemäß einem der Ansprüche 1-6, dadurch gekennzeichnet, daß

a) für X = $-SO_2-$ entsprechend Schema 1 verfahren wird.

Sulfonsäure-1H-indazol-3-ylester **II** werden in Gegenwart einer Base und gegebenenfalls in Gegenwart eines Verdünnungsmittels zu Verbindungen der allgemeinen Formel **III** umgesetzt, wobei $R^1$, $R^3$, X und Z die oben genannte Bedeutung besitzen.

Sulfonsäure-1H-indazol-3-ylester **II** oder 1-Sulfonyl-indazole **III** werden gegebenenfalls in Gegenwart einer Base, insbesondere Natriumhydrid, und gegebenenfalls in Gegenwart eines Verdünnungsmittels, insbesondere Dimethylsulfoxid, mit Verindungen der allgemeinen Formel Hal-Y-$R^2$ wobei $R^1$, $R^2$, $R^3$, X, Y und Z die oben genannte Bedeutung besitzen und Hal ein Halogenatom F, Cl, Br oder Jod bedeutet, zu Verbindungen der allgemeinen Formel **I** umgesetzt, wobei $R^1$, $R^2$, $R^3$, X, Y und Z die oben genannte Bedeutung besitzen.

Schema 1:

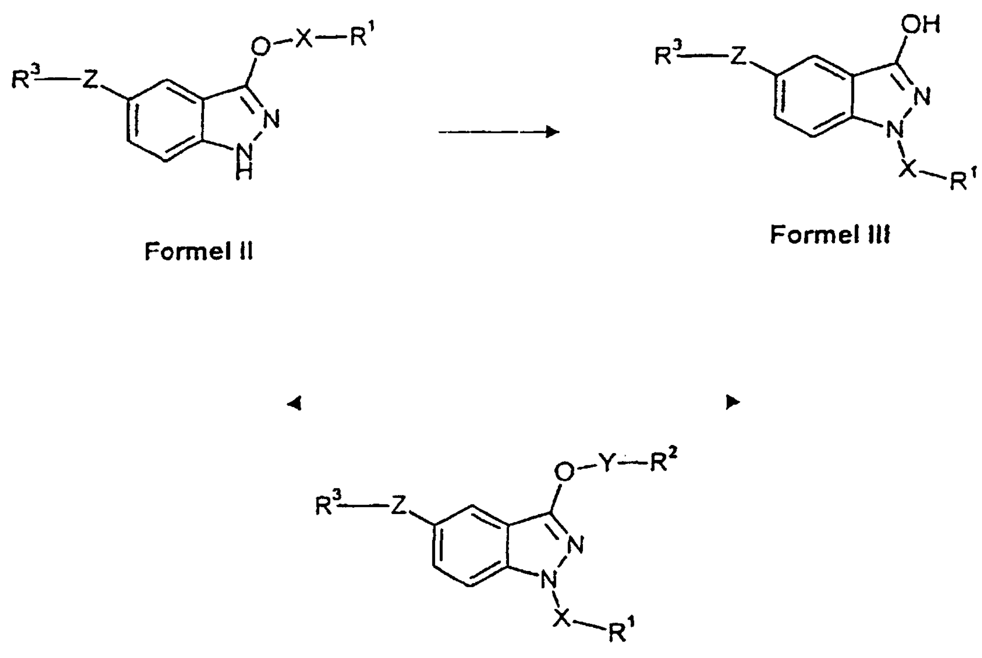

Formel II

Formel III

Formel I

b) für X = $-(CH_2)_p-$, $-(CH_2)_p-O-$,
mit p = 1...4 entsprechend Schema 2 verfahren wird.

Verbindungen der allgemeinen Formel III werden gegebenenfalls in Gegenwart einer Base, insbesondere Natriumhydrid, und gegebenenfalls in Gegenwart eines Verdünnungsmittels, insbesondere Dimethylsulfoxid, mit Verbindungen der allgemeinen Formel Hal-Y-$R^2$ wobei $R^1$, $R^2$, $R^3$, X, Y und Z die oben genannte Bedeutung besitzen und Hal ein Halogenatom F, Cl, Br oder Jod bedeutet, zu Verbindungen der allgemeinen Formel **I** umgesetzt, wobei $R^1$, $R^2$, $R^3$, X, Y und Z die oben genannte Bedeutung besitzen.

Schema 2:

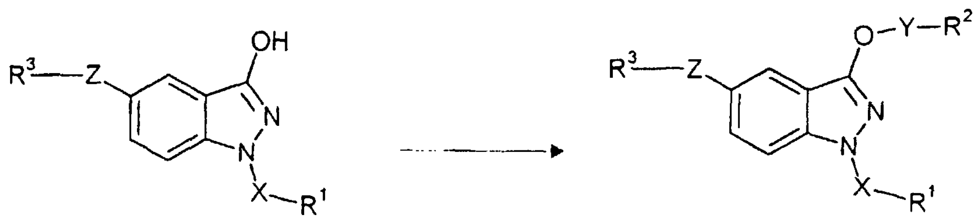

**Formel III** **Formel I**

wobei **Formel III** auch als tautomere Fom **Formel IV** entsprechend Schema 3 vorliegen kann.

Schema 3:

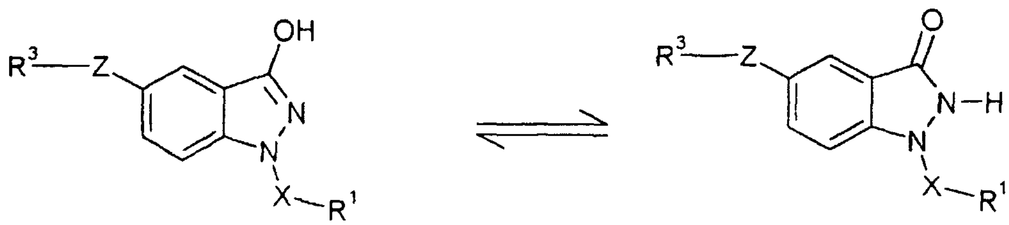

**Formel III** **Formel IV**

Die Verbindungen der allgemeinen Formel **I** sind neu.

Ausführungsbeispiele

Von den erfindungsgemäßen Verbindungen werden folgende Vertreter beispielhaft genannt:

3-[2-(2-Brom-4,6-difluorphenoxy)ethoxy]-5-methoxy-1-(toluol-4-sulfonyl)-1H-indazol 5-Methoxy-1-(toluol-4-sulfonyl)-3-[5-(3-trifluormethylphenyl)-1,2,4-oxadiazol-3-ylmethoxy]-1H-indazol
6-{2-[5-Methoxy-1 -(toluol-4-sulfonyl)-1H-indazol-3-yloxy]acetyl}-3,4-dihydro-1Hchinolin-2-on
N-(2,4-Difluorphenyl)-2-[5-methoxy-1-(toluol-4-sulfonyl)-1H-indazol-3-yloxy]-acetamid
3-(6-Chlorbenzo [1,3] dioxol-5-ylmethoxy)-5-methoxy-1-(3-nitrobenzyl)-1H-indazol
3-[3-(4-Fluorphenyl)-propoxy]-5-nitro-1-(3-nitrobenzyl)-1H-indazol
3-[3-(6-Chlorbenzo [1,3] dioxol-5-ylmethoxy)-5-methoxy-indazol-1-ylmethyl]-phenylamin
1-(4-Fluorbenzyl )-5-methoxy-3-[2-(4-nitrophenoxy)ethoxy]-1H-indazol
3-[2-(2-Brom-4,6-difluorphenoxy)ethoxy]-5-methoxy-1-[2-(4-nitrophenoxy)ethyl]-1H-indazol
3-[2-(2-Brom-4,6-difluorphenoxy)ethoxy]-1-[3,4-dichlorbenzyl]-5-methylthio-1H-indazol
1-{1-(2,4-Dichlorbenzyl)-3-[2-(4-nitrophenoxy)ethoxy]-1H-indazol-5-yl}-3-naphthalen-1-ylharnstoff

Die Charakterisierung der Verbindungen erfolgte mittels Schmelzpunkt, Dünnschicht-chromatographie, Elementaranalyse, NMR-Spektroskopie, IR-und UV/VIS-Spektroskopie und gegebenfalls mit Massenspektroskopie.

Reinigung mit Säulenflüssigkeitschromatographie:

Bei der Herstellung der Verbindungen Beispiel 1 bis 123 können als Nebenprodukte die 1,2-Dihydro-indazol-3-one gemäß der allgemeinen Formel **V** gebildet werden.

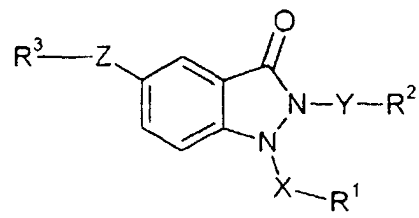

**Formel V**

Die Verbindungen der allgemeinen Formel **I** lassen sich gewöhnlich durch Umkristallisation von den Verbindungen der allgemeinen Formel **V** trennen. Gelingt dies nicht, ist eine säulenchromatographische Trennung unter folgenden Bedingungen erforderlich: Stationäre Phase: Normalphasen-Kieselgel, z.B. Si 60 bis 100 Å, Partikelgröße 5 bis 100 µM. Eluent: Methylenchlorid / Ethylacetat = 95 / 5 oder Methylenchlorid / Methanol = 95 / 5.
Die Verbindungen der allgemeinen Formel **V** sind polarer als die Verbindungen der allgemeinen Formel **I,** so daß die Verbindungen der allgemeinen Formel **I** unter diesen chromatographischen Bedingungen zuerst eluiert werden. Diese Reinigungsoperation ist für alle Beispiele 1 bis 123 anwendbar.

Beispiel 1

3-[2-(2-Brom-4,6-difluorphenoxy)ethoxy]-5-methoxy-1-(toluol-4-sulfonyl)-1H-indazol

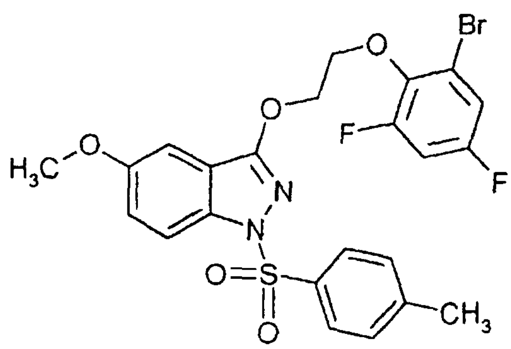

31,8 g (100 mmol) Toluol-4-sulfonsäure-5-methoxy-1H-indazol-3-ylester werden in 300 ml DMSO gelöst und portionsweise mit 3,54 g (140 mmol) Natriumhydrid (95proz.) versetzt. Nach 15 Minuten Rühren wird eine Lösung von 28,5 g (105 mmol) 1-(2-Brom-4,6-difluorphenoxy)-2-chlor-ethan in 100 ml DMSO zugetropft und 3 Stunden bei 90°C gerührt. Nach dem Erkalten wird in 1,5 l Wasser eingerührt, dreimal mit je 400 ml Ethylacetat extrahiert, die organische Phase über Natriumsulfat getrocknet, im Vakuum zur Trockne destilliert und der Rückstand aus Ethanol umkristallisiert.
Ausbeute: 25,1 g (45,3 % der Theorie)
F. 133-134,5 °C
$^{13}C$-NMR (DMSO-$d_6$; 300 MHz): $\delta$ = 21,3 $CH_3$; 55,9 $CH_3O$; 69,3 und 72,1 je $CH_2O$.

Als Ausgangsprodukte zur Herstellung der Beispiele 2 bis 76 (Tabellen 1,2,3 und 4) wurden folgende Produkte verwendet:

Toluol-4-sulfonsäure-5-methoxy-1H-indazol-3-ylester
4-Chlor-benzolsulfonsäure-5-methoxy-1H-indazol-3-ylester
4-Fluor-benzolsulfonsäure-5-methoxy-1H-indazol-3-ylester
4-Methoxy-benzolsulfonsäure-5-methoxy-1H-indazol-3-ylester
4-Trifluormethoxy-benzolsulfonsäure-5-methoxy-1H-indazol-3-ylester
Thiophen-2-sulfonsäure-5-methoxy-1H-indazol-3-ylester
4-Acetylamino-benzolsulfonsäure-5-methoxy-1H-indazol-3-ylester
Chinolin-8-sulfonsäure-5-methoxy-1H-indazol-3-ylester
Naphthalin-1-sulfonsäure-5-methoxy-1H-indazol-3-ylester
2,5-Dichlor-benzolsulfonsäure-5-methoxy-1H-indazol-3-ylester

4-(5-Methoxy-1H-indazol-3-yloxysulfonyl)-benzoesäure
Toluol-4-sulfonsäure-5-nitro-1H-indazol-3-ylester
4-Methoxy-benzolsulfonsäure-5-nitro-1H-indazol-3-ylester

Die Synthese der Verbindungen Beispiel 2 bis 31 gelingt analog der Vorschrift nach Beispiel 1. Als weitere Ausgangsprodukte werden entsprechend $R^3$ und n substituierte Phenoxyalkylbromide bzw. -chloride verwendet.

Tabelle 1: 3-Phenoxyalkyloxy-indazol-1-sulfonamide

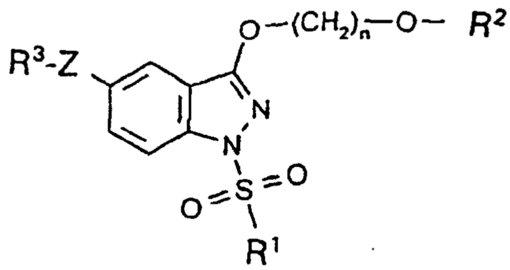

Formel VI

$R^3$-Z wobei X = $-SO_2-$ und Y = $-(CH_2)_n-O-$

| Beispiel | $R^1$ | $R^2$ | n | Ausbeute (% d.Th.) | F. [°C] | $^{13}$C-NMR (DMSO-$d_6$) $CH_2$ [ppm] |
|---|---|---|---|---|---|---|
| 2 | 4-Tolyl | Phenyl | 2 | 37 | 140 (2-PrOH) | 65,22; 68,26 |
| 3 | 4-Tolyl | Phenyl | 3 | 29 | 113-115 (2-PrOH) | 28,51; 64,24; 66,99 |
| 4 | 4-Tolyl | Phenyl | 4 | 22 | 136-137 (2-PrOH) | 25,37; 25,60; 67,32; 69,75 |
| 5 | 4-Tolyl | 2-Bromphenyl | 2 | 6 | 123-125 (MeOH) | 64,92; 66,24 |
| 6 | 4-Tolyl | 4-Chlorphenyl | 2 | 26 | 153 (2-PrOH) | 64,73; 67,13 |
| 7 | 4-Chlor-phenyl | 4-Chlorphenyl | 2 | 9 | 149-154 (MeCN) | 66,24; 68,74 |
| 8 | 4-Fluor-phenyl | 4-Chlorphenyl | 2 | 29 | 121-126 (EtOH) | 65,48; 67,97 |
| 9 | 4-Methoxy-phenyl | 4-Chlorphenyl | 2 | 37 | 160-162 (2-PrOH) | 66,26; 68,61 |
| 10 | 4-Trifluor-methoxy-phenyl | 4-Chlorphenyl | 2 | 22 | 117-120 (MeCN) | 66,24; 68,77 |
| 11 | 4-Tolyl | 2,6-Dichlor-phenyl | 2 | 34 | 136-137,5 (MeCN) | 69,24; 71,42 |
| 12 | 4-Tolyl | 2,4-Difluor-phenyl | 2 | 15 | 96-100 (EtOAc) | 67,87; 68,69 |
| 13 | 4-Tolyl | 2,6-Difluor-phenyl | 2 | 21 | 101-102,5 (EtOH) | 69,35; 72,20 |
| 14 | 4-Tolyl | 2,4,6-Trifluor-phenyl | 2 | 7 | 110-111,5 (EtOH) | 69,29; 72,45 |
| 15 | 4-Methoxy-phenyl | 2-Brom-4,6-difluor-phenyl | 2 | 10 | 119-121,5 (EtOAc) | 67,64; 70,49 |

| | | | | | |
|---|---|---|---|---|---|
| 16 | 4-Chlor-phenyl | 2-Brom-4,6-difluor-phenyl | 2 5 | 138-139,5 (EtOH) | 69,41; 72,05 |
| 17 | 4-Tolyl | 2,6-Dibrom-4-nitro-phenyl | 2 16 | 131 (MeCN) | 69,17; 72,17 |
| 18 | 4-Methoxy-phenyl | 2,6-Dibrom-4-nitro-phenyl | 2 14 | 153 (EtOH) | 69,72; 72,73 |
| 19 | 4-Tolyl | 4-Nitrophenyl | 2 9 | 152-156 (2-PrOH) | 66,92; 68,40 |
| 20 | 4-Chlor-phenyl | 4-Nitrophenyl | 2 16 | 159-161 (MeCN) | 66,91; 68,49 |
| 21 | 4-Methoxy-phenyl | 4-Nitrophenyl | 2 20 | 174-178 (2-PrOH) | 66,96; 68,37 |
| 22 | 2-Thiophenyl | 4-Nitrophenyl | 2 35 | 150-155 (MeOH) | 66,93; 69,58 |
| 23 | 4-Tolyl | 4-Cyanophenyl | 2 19 | 148-151 (2-PrOH) | 65,64; 67,65 |
| 24 | 4-Tolyl | 4-Carboxamido-phenyl | 2 15 | 175-180 (2-PrOH) | 66,03; 68,62 |

Beispiel 32

5-Methoxy-1-(toluol-4-sulfonyl)-3-[5-(3-trifluormethylphenyl)-1,2,4-oxadiazol-3-ylmethoxy]-1H-indazol

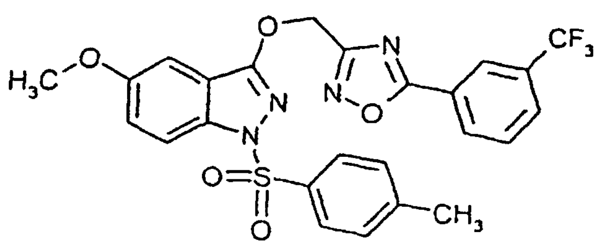

3,06 g (9,5 mmol) Toluol-4-sulfonsäure-5-methoxy-1H-indazol-3-ylester werden in 50 ml DMSO gelöst und portionsweise mit 0.34 g (14.2 mmol) Natriumhydrid (95proz.) versetzt. Nach 15 Minuten Rühren wird eine Lösung von 2,5 g (9,5 mmol) 3-Chlormethyl-5-[3-(trifluormethyl)phenyl]-1,2,4-oxadiazol in 20 ml DMSO zugetropft und 3 Stunden bei 60°C gerührt. Nach dem Erkalten wird in 200 ml Wasser eingerührt, 6 Stunden gerührt, der Niederschlag abgesaugt und aus Ethanol umkristallisiert.

Ausbeute: 1,9 g (36,7 % der Theorie)

F. 138-144 °C

$^{13}$C-NMR (DMSO-$d_6$; 300 MHz): $\delta$ = 19,0 $CH_3$; 54,1 $CH_3O$; 60,0 $CH_2O$.

Die Synthese der Verbindungen Beispiel 33 bis 50 gelingt analog der Vorschrift nach Beispiel 32.

Tabelle 2: 3-Benzyloxy-indazol-1-sulfonamide oder Heteroanaloge

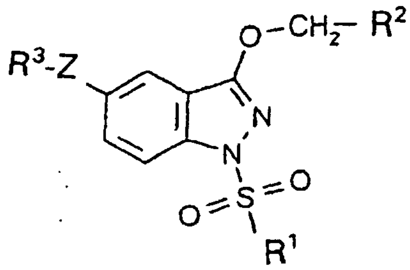

**Formel VII**

$R^3$-Z wobei X = $-SO_2-$ und Y = $-CH_2-$

| Beispiel | $R^1$ | $R^2$ | Ausbeute (% d.Th.) | F. [°C] | $^{13}C$-NMR ($DMSO-d_6$) $CH_2O$ [ppm] |
|---|---|---|---|---|---|
| 33 | 4-Tolyl | 4-Benzyloxy-phenyl | 23 | 159-161 (MeCN) | 70,51; 71,52 |
| 34 | 4-Tolyl | 2-Methoxy-5-acetylphenyl | 15 | 132-136 (EtOH) | 65,67 |
| 35 | 4-Tolyl | 2-Fluor-6-chlor-phenyl | 21 | 156-160 (2-PrOH) | 61,76 |
| 36 | 4-Chlorphenyl | 2-Fluor-6-chlor-phenyl | 23 | 151-155 (EtOH) | 62,78 |

| | | | | | |
|---|---|---|---|---|---|
| 37 | 4-Fluorphenyl | 3-Trifluormethylphenyl | 10 | 113 (MeOH) | 70,48 |
| 38 | 4-Tolyl | 2-Fluorphenyl | 23 | 151-152 (2-PrOH) | 65,43 |
| 39 | 4-Fluorphenyl | 2-Fluorphenyl | 18 | 149-150 (MeCN) | 66,39 |
| 40 | 4-Fluorphenyl | 4-Fluorphenyl | 9 | 110-114 (EtOH) | 69,16 |
| 41 | 4-Tolyl | 4-Chlor-2-nitrophenyl | 25 | 85-90 (2-PrOH) | 67-74 |
| 42 | 4-Tolyl | 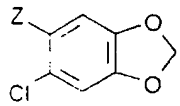 | 34 | 198-202 (MeCN) | 68.68; 102,66 |
| 43 | 4-Tolyl | 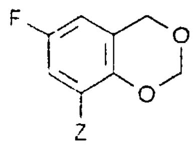 | 19 | 183-184 (EtOAc) | 65,35; 65,63; 91,49 |
| 44 | 4-Tolyl | 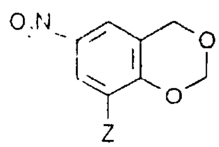 | 10 | 248-252 (MeCN) | 65,25; 65,79; 92,38 |
| 45 | 4-Fluorphenyl | 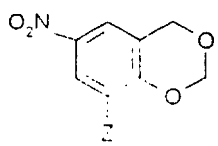 | 10 | 246-250 (MeCN) | 64,55; 65,93; 91,58 |
| 46 | 4-Tolyl | 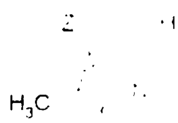 | 10 | 168-174 (EtOH) | 62,82 |
| 47 | 4-Chlorphenyl | 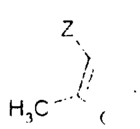 | 14 | 180-182 (EtOH) | 60,92 |
| 48 | 4-Tolyl | 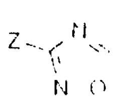 | 41 | 153-157 (MeCN) | 61,27 |

| | | | | | |
|---|---|---|---|---|---|
| 49 | 4-Tolyl | 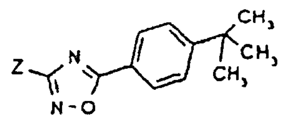 | 5 | 172-174 (EtOH) | 61,34 |
| 50 | 4-Tolyl | 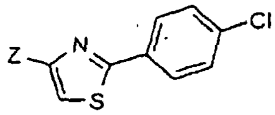 | 23 | 180-181 (MeCN) | 67,77 |

In den Beispielen 42-48 bedeutet Z die Verknüpfungsstelle der Reste $R^2$ mit der Formel VII.

Beispiel 51

6-{2-[5-Methoxy-1-(toluol-4-sulfonyl)-1H-indazol-3-yloxy]acetyl}-3,4-dihydro-1H-chinolin-2-on

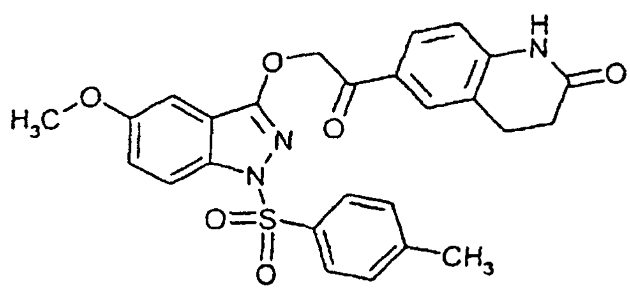

3,66 g (11,5 mmol) Toluol-4-sulfonsäure-5-methoxy-1H-indazol-3-ylester werden in 50 ml DMSO gelöst und portionsweise mit 0,41 g (17,2 mmol) Natriumhydrid (95proz.) versetzt. Nach 15 Minuten Rühren wird eine Lösung von 3,16 g (11,5 mmol) 6-(Bromacetyl)-2-oxo-1,2,3,4-tetrahydro-chinolin in 30 ml DMSO zugetropft und 3 Stunden bei 60°C gerührt. Nach dem Erkalten wird in 200 ml Wasser eingerührt, 6 Stunden gerührt, der Niederschlag abgesaugt und aus Acetonitril umkristallisiert.
Ausbeute: 2,2 g (37,8 % der Theorie)
F. 232-237 °C
$^{13}$C-NMR (DMSO-$d_6$; 300 MHz): δ = 21,4 $CH_3$; 23,2 $CH_2$; 28,7 $CH_2$ ; 56,1 $CH_3O$; 69,5 $CH_2O$; 189,3 2 x C=O.
IR (KBr): 1680 C=O.

Die Synthese der Verbindungen Beispiel 52 bis 65 gelingt analog der Vorschrift nach Beispiel 51.

Tabelle 3: 3-Phenacyl-indazol-1-sulfonamide

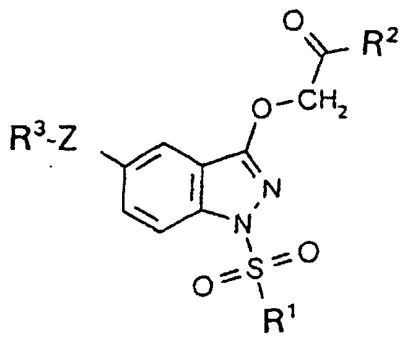

**Formel VIII**

$R^3$-Z wobei X = $-SO_2-$ und Y = $-O-CH_2-(C=O)-$

| Beispiel | $R^1$ | $R^2$ | Ausbeute (% d.Th.) | F. [°C] | $^{13}$C-NMR (DMSO-$d_6$) $CH_2O$ / C=O |
|---|---|---|---|---|---|
| 52 | 4-Tolyl | 4-Chlor-phenyl | 7 | 173-180 (MeCN) | 70,00 / 190,55 |
| 53 | 4-Methoxy-phenyl | 4-Chlor-phenyl | 37 | 183-183,5 (MeCN) | 70,59 / 191,38 |
| 54 | 4-Chlorphenyl | 4-Chlor-phenyl | 21 | 166-169 (2-PrOH) | 72,50 / 192,84 |
| 55 | 4-Tolyl | 3,4-Dichlor-phenyl | 9 | 173-178 (MeCN) | 72,65 / 192,52 |
| 56 | 4-Chlorphenyl | 3,4-Dichlor-phenyl | 13 | 175-178 (MeCN) | 70,42 / 190,03 |
| 57 | 4-Acetyl-aminophenyl | 3,4-Dichlor-phenyl | 4 | 225-229 (EtOH) | 71,51 / 191,47 |
| 58 | Naphthalen-1-yl | 3,4-Dichlor-phenyl | 17 | 210-212 (MeCN) | 70,97 / 190,68 |
| 59 | Chinolin-8-yl | 3,4-Dichlor-phenyl | 33 | 213-218 (MeCN) | 71,19 / 191,43 |

| | | | | | |
|---|---|---|---|---|---|
| 60 | 4-Tolyl | 4-Diphenyl | 8 | 196-200 (MeCN) | 73,54 / 194,35 |
| 61 | 4-Chlorphenyl | 4-Diphenyl | 44 | 207-210 (MeCN) | 73,04 / 193,65 |
| 62 | 4-Methoxy-phenyl | 4-Diphenyl | 23 | 198-205 MeCN) | 71,55 / 192,43 |
| 63 | 4-Acetyl-aminophenyl | 4-Diphenyl | 2 | 248-249 (n-BuOH) | 71,53 / 192,47 |

Beispiel 66

N-(2,4-Difluorphenyl)-2-[5-methoxy-1-(toluol-4-sulfonyl)-1H-indazol-3-yloxy]-acetamid

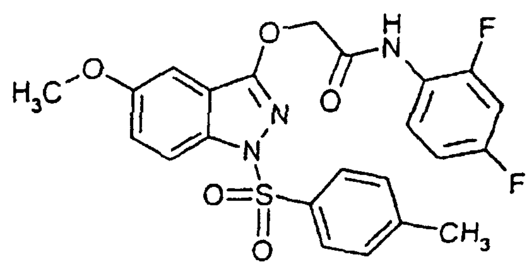

3,66 g (11,5 mmol) Toluol-4-sulfonsäure-5-methoxy-1H-indazol-3-ylester werden in 50 ml DMSO gelöst und portionsweise mit 0,41 g (17,2 mmol) Natriumhydrid (95proz.) versetzt. Nach 15 Minuten Rühren wird eine Lösung von 2,36 g
(11,5 mmol) α-Chlor-2,4-difluoracetanilid in 20 ml DMSO zugetropft und 3 Stunden bei 60°C gerührt. Nach dem Erkalten wird in 200 ml Wasser eingerührt, 6 Stunden gerührt, der Niederschlag abgesaugt und aus Methanol umkristallisiert. Der auskristallisierte Niederschlag wird abgesaugt (Nebenprodukt), das Filtrat auf 30 ml eingeengt und nach beendeter Kristallisation abgesaugt.
Ausbeute: 1,0 g (17,8 % der Theorie)
F. 155-159 °C
$^{13}$C-NMR (DMSO-$d_6$; 300 MHz): δ = 20,8 $CH_3$; 55,7 $CH_3O$; 67,2 $CH_2O$; 165,4 C=O.
IR (KBr): 1706 C=O.

Die Synthese der Verbindungen Beispiel 67 bis 76 gelingt analog der Vorschrift nach Beispiel 66.

Tabelle 4: Andere 3-subst. Indazol-1-sulfonamide

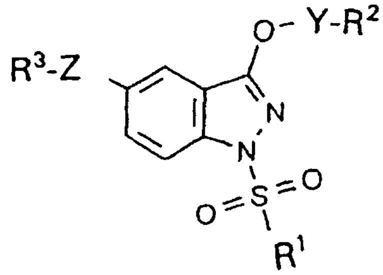

Formel IX

$R^3$-Z = $CH_3O$

| Beispiel | $R^1$ | -Y-$R^2$ | Ausbeute (% d.Th.) | F. [°C] | $^{13}$C-NMR ($DMSO-d_6$) $CH_2O$ [ppm] |
|---|---|---|---|---|---|
| 67 | 4-Tolyl | 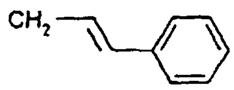 | 16 | 128-129 ( 2-PrOH)) | 70,13 |
| 68 | 4-Tolyl | 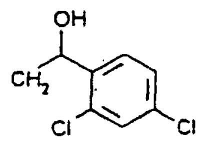 | 5 | 135-140 (EtOH) | 72,79 |
| 69 | 4-Tolyl | 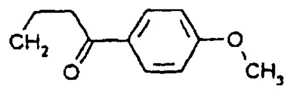 | 18 | 142-144 (EtOH) | 67,08 |
| 70 | 4-Fluor-phenyl | 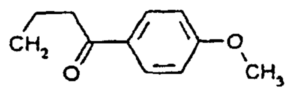 | 6 | 154-157 (MeCN) | 69,58 |
| 71 | 4-Tolyl | 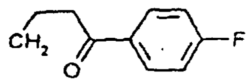 | 5 | 165-169 (MeOH) | 69,40 |
| 72 | 4-Tolyl | 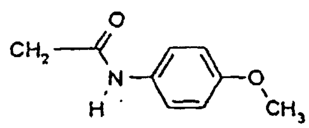 | 29 | 177-178,5 (EtOH) | 67,97 |
| 73 | 4-Tolyl | 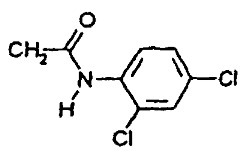 | 39 | 196-200 (MeCN) | 67,79 |
| 74 | 4-Tolyl | 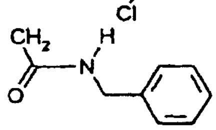 | 24 | 147-151 (EtOH) | 65,86 |
| 75 | 4-Tolyl | 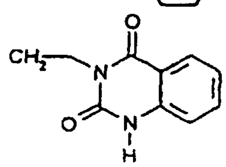 | 14 | 238-241 (MeCN) | 67,20 |
| 76 | 4-Meth-oxyphenyl | 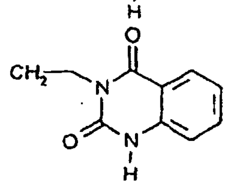 | 24 | 216 Zers. MeCN) | 66,85 |

Beispiel 77

3-(6-Chlorbenzo [1,3] dioxol-5-ylmethoxy)-5-methoxy-1-(3-nitrobenzyl)-1H-indazol

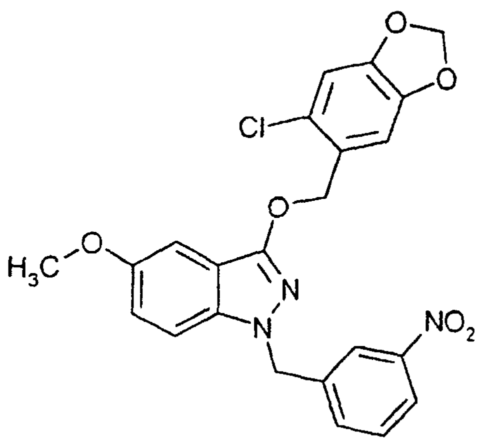

4,5 g (15 mmol) 5-Methoxy-1-(3-nitrobenzyl)-1H-indazol-3-ol werden in 100 ml DMSO gelöst und portionsweise mit 0,72 g (18 mmol) Natriumhydrid (60proz.) versetzt. Nach 2 Stunden Rühren wird eine Lösung von 3,1 g (15 mmol) 6-Chlorpiperonylchlorid in 20 ml DMSO zugetropft und 3 Stunden bei 60°C gerührt. Nach dem Erkalten werden 250 ml Wasser zugetropft, 4 Stunden gerührt und abgesaugt. Der Niederschlag wird zunächst aus Isopropanol, anschließend aus Ethanol umkristallisiert.
Ausbeute: 3,3 g (47,0 % der Theorie)
F. 134-135,5 °C
$^{13}$C-NMR (DMSO-$d_6$ ; 300 MHz): $\delta$ = 51,5 $CH_2N$; 55,7 $CH_3O$; 67,8 $CH_2O$; 101,7 $OCH_2O$.

Die Synthese der Verbindungen Beispiel 78 bis 85 gelingt analog der Vorschrift nach Beispiel 77.

Tabelle 5: 1-(3-Nitrobenzyl)-3-alkyloxy-indazole

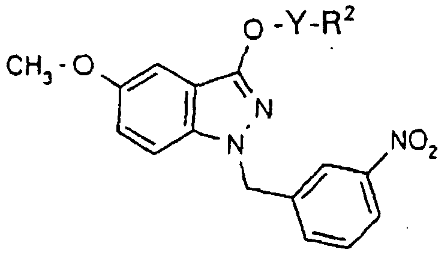

**Formel XI**

| Beispiel | $-Y-R^2$ | Ausbeute (% d.Th.) | F. [°C] | $^{13}C$-NMR ($DMSO-d_6$) $N-CH_2$/ $O-CH_2$ |
|---|---|---|---|---|
| 78 | 4-Chlorphenoxyethyl | 31 | 118-120 (2-PrOH) | 51,46/ 66,68; 67,32 |
| 79 | 4-Nitrophenoxyethyl | 75 | 163-166 (Aceton) | 50,48/ 67,03; 67,10 |
| 80 | 4-Carboxamino-phenoxyethyl | 94 | 159-162 (EtOH) | 50,47/ 66,22; 67,28 |
| 81 | 2-Brom-4,6-difluorphenoxyethyl | 44 | 63-73 (2-PrOH) | 52,01/ 68,32; 72,80 |
| 82 | 2,6-Dibrom-4-nitrophenoxyethyl | 18 | 118-121 (EtOAc) | 51,95/ 68,09; 72,59 |
| 83 | 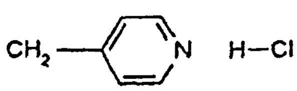 | 55 | 160-170 (Aceton) | 50,39/ 68,30 |
| 84 | 2,6-Dichlorbenzyl | 61 | 139-143 (EtOH) | 50,49/ 65,31 |
| 85 | 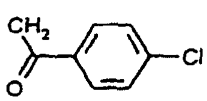 | 53 | 141-144 (2-PrOH) | 51,91/ 71,00 |

Beispiel 87

3-[3-(6-Chlorbenzo [1,3] dioxol-5-ylmethoxy)-5-methoxy-indazol-1-ylmethyl]-phenylamin

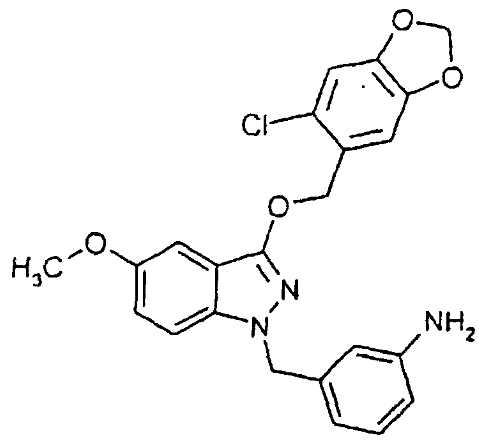

2,2 g (4,7 mmol) 3-(6-Chlorbenzo [1,3] dioxol-5-ylmethoxy)-5-methoxy-1-(3-nitrobenzyl)-1H-indazol werden in 500 ml Dioxan gelöst, mit ca. 1 g Raney-Nickel versetzt und bei 70°C, 20 bar, 2 Stunden hydriert. Nach dem Erkalten wird der Katalysator abgesaugt, im Vakuum zur Trockne destilliert und der Rückstand aus Dioxan umkristallisiert.
Ausbeute: 1,8 g (87,5 % der Theorie)
F. 153-154 °C
$^{13}$C-NMR (DMSO-$d_6$ ; 300 MHz): $\delta$ = 52,6 $CH_2N$; 55,7 $CH_3O$; 67,9 $CH_2O$; 101,7 $OCH_2O$.

Die Synthese der Verbindungen Beispiel 88 bis 90 gelingt analog der Vorschrift nach Beispiel 87.

Tabelle 6: 1-(3-Aminobenzyl)-3-alkyloxy-indazole

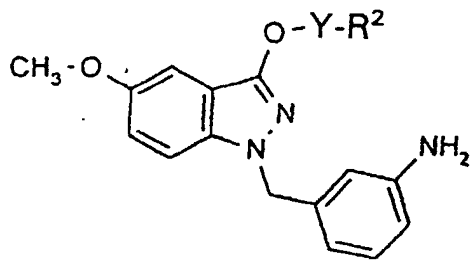

Formel XII

| Beispiel | $-Y-R^2$ | Ausbeute (% d.Th.) | F. [°C] | $^{13}C$-NMR ($DMSO-d_6$) $N-CH_2/O-CH_2$ |
|---|---|---|---|---|
| 88 | 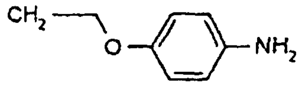 2 H—Cl | 89 | 227-230 (2-PrOH) | 50,99/ 66,63; 67,35 |
| 89 | 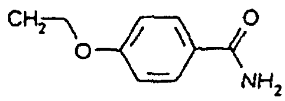 | 77 | 176-178 (Dioxan) | 52,00/ 66,43; 67,33 |
| 90 | 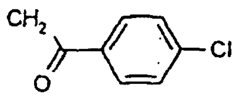 H—Cl | 40 | 155-158 (2-PrOH) | 50,84/ 70,57 |

Beispiel 91

1-(4-Fluorbenzyl)-5-methoxy-3-[2-(4-nitrophenoxy)ethoxy]-1H-indazol

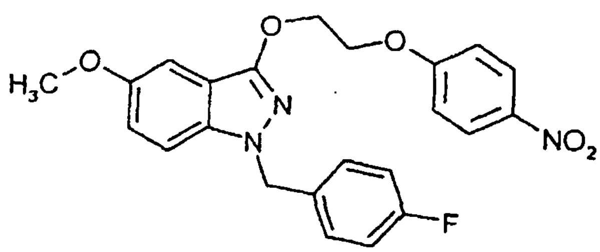

4,1 g (15 mmol) 1-(4-Fluorbenzyl)-5-methoxy-1H-indazol-3-ol werden in 100 ml DMSO gelöst und portionsweise mit 1 g (25 mmol) Natriumhydrid (60proz.) versetzt. Nach 2 Stunden Rühren wird eine Lösung von 3,7 g (15 mmol) 2-(4-Nitrophenoxy)-ethylbromid in 20 ml DMSO zugetropft und 3 Stunden bei 80-90°C gerührt. Nach dem Erkalten werden 250 ml Wasser zugetropft, 4 Stunden gerührt, abgesaugt und aus Ethanol umkristallisiert.
Ausbeute: 3,1 g (47,2 % der Theorie)
F. 117-120,5 °C
$^{13}C$-NMR ($DMSO-d_6$; 300 MHz): $\delta$ = 50.7 $CH_2N$; 55,4 $CH_3O$; 67,0 $CH_2O$; 67,2 $CH_2O$.

Die Synthese der Verbindungen Beispiel 92 bis 98 gelingt analog der Vorschrift nach Beispiel 91.

Tabelle 7: 1-(4-Fluorbenzyl)-3-alkyloxy-indazole

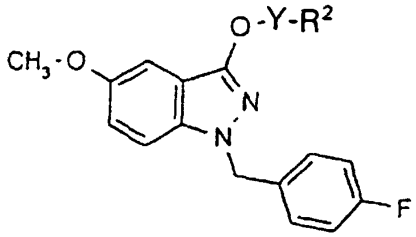

**Formel XIII**

| Beispiel | -Y-$R^2$ | Ausbeute (% d.Th.) | F. [°C] | $^{13}$C-NMR ($DMSO-d_6$) $N-CH_2$/ $O-CH_2$ |
|---|---|---|---|---|
| 92 | 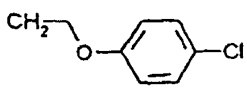 | 48 | 81,5-83,5 (EtOH) | 50,51/ 65,49; 65,99 |
| 93 | 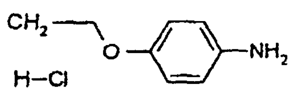 | 75 | 190-195 (2-PrOH) | 50,73/ 66,52; 67,21 |
| 94 | 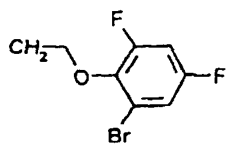 | 40 | 64-66 (2-PrOH) | 52,22/ 68,27; 72,93 |
| 95 | 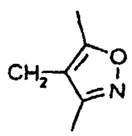 | 84 | 97-98 (EtOH) | 52,25/ 60,10 |
| 96 | 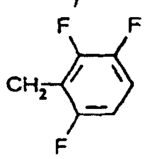 | 73 | 72-75 (EtOH) | 51,96/ 58,57 |
| 97 | 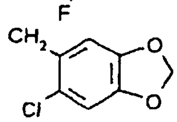 | 75 | 135-136,5 (2-PrOH) | 52,31/ 68,32 |
| 98 | 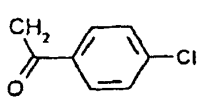 | 73 | 119-121 (EtOH) | 53,79/ 72,73 |

<u>Beispiel 99</u>

3-[2-(2-Brom-4,6-difluorphenoxy)ethoxy]-5-methoxy-1-[2-(4-nitrophenoxy)ethyl]-1Hindazol

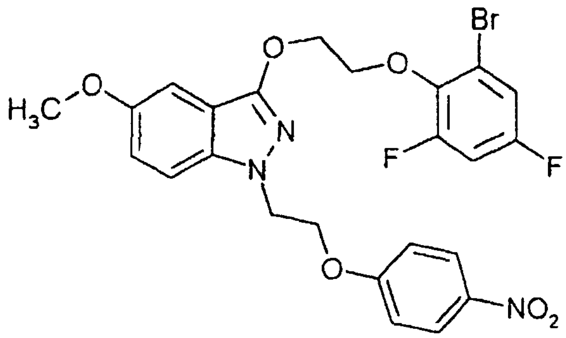

2,7 g (8 mmol) 5-Methoxy-1-[2-(4-nitrophenoxy)ethyl]-1H-indazol-3-ol werden in 50 ml DMSO gelöst und portionsweise mit 0,4 g (16,7 mmol) Natriumhydrid (95proz.) versetzt. Nach 15 Minuten Rühren wird eine Lösung von 2,17 g (8 mmol) 1-(2-Brom-4,6-difluorphenoxy)-2-chlorethan in 20 ml DMSO zugetropft und 3 Stunden bei 60°C gerührt. Nach dem Erkalten werden 200 ml Wasser zugegeben, 6 Stunden gerührt, abgesaugt und aus Ethanol umkristallisiert.
Ausbeute: 1,7 g (37,6 % der Theorie)
F. 102 °C
$^{13}C$-NMR (DMSO-$d_6$; 300 MHz): $\delta$ = 46,3 $CH_2N$; 54,3 $CH_3O$; 66,6 $CH_2O$; 66,9 $CH_2O$; 71,3 $CH_2O$.

Die Synthese der Verbindungen Beispiel 100 bis 111 gelingt analog der Vorschrift nach Beispiel 99.

Tabelle 8: 1-(Phenoxyethyl)-3-alkyloxy-indazole

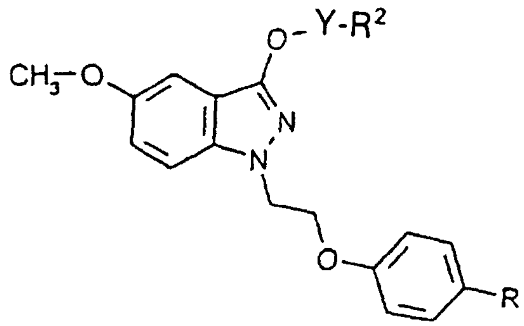

**Formel XIV**

R = $NO_2$

| Beispiel | $-Y-R^2$ | Ausbeute (% d.Th.) | F. [°C] | $^{13}C$-NMR ($DMSO-d_6$) $O-CH_2$ |
|---|---|---|---|---|
| 100 | 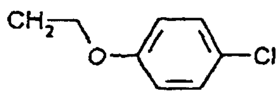 | 48 | 110-116 (EtOH) | 67,86; 68,55; 68,84 |
| 101 | 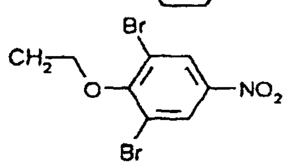 | 18 | 157-161 (EtOAc) | 65,90; 66,06; 70,53 |
| 102 | 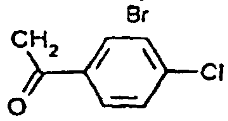 | 33 | 181-184 (MeCN) | 68,21; 71,36 |
| 103 | 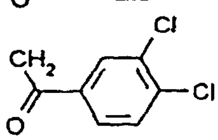 | 29 | 195-197 (MeCN) | 66,91; 70,44 |
| 104 | 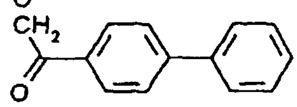 | 63 | 153-156 (MeCN) | 67,71; 70,89 |
| 105 | 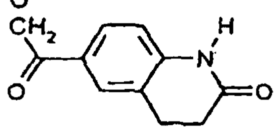 | 50 | 158-165 (MeCN) | 67,78; 70,60 |
| 106 | 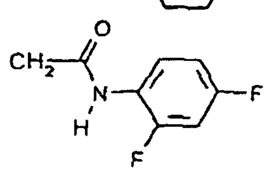 | 40 | 167-171 (MeCN) | 67,51; 67,03 |

| | | | | |
|---|---|---|---|---|
| 107 | 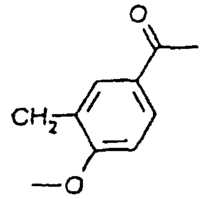 | 55 | 91-98 (EtOH) | 64,69; 66,97 |
| 108 | 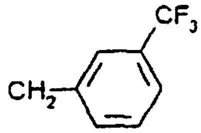 | 47 | 91-96 (EtOH) | 67,87; 69,65 |
| 109 | 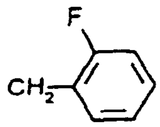 | 60 | 144-147 (MeCN) | 64,50; 67,89 |
| 110 | 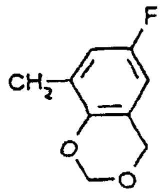 | 53 | 177-178,5 (MeCN) | 63,93; 64,82; 67,04; 90,63 |

**Formel XIV**, R = Cl

| Beispiel | -Y-$R^2$ | Ausbeute (% d.Th.) | F. [°C] | $^{13}$C-NMR ($DMSO-d_6$) $O-CH_2$ |
|---|---|---|---|---|
| 111 | 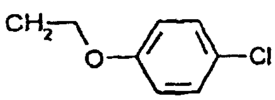 | 6 | 110-116 (EtOH) | 66,92; 67,32; 67,60 |

Beispiel 112

3-[2-(2-Brom-4,6-difluorphenoxy)ethoxy]-1-[3,4-dichlorbenzyl]-5-methylthio-1Hindazol

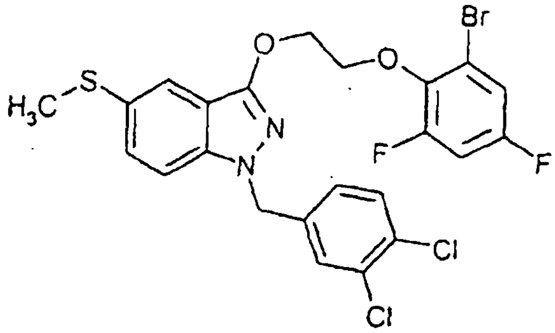

3,6 g (11 mmol) 1-(3,4-Dichlorbenzyl)-5-methylthio-1H-indazol-3-ol werden in 100 ml DMSO gelöst und portionsweise mit 0,34 g (13,2 mmol) Natriumhydrid (95proz.) versetzt. Nach 2 Stunden Rühren wird eine Lösung von 3,0 g (11 mmol) 1-(2-Brom-4,6-difluorphenoxy)-2-chlorethan in 20 ml DMSO zugetropft und 3 Stunden bei 60°C gerührt. Nach dem Erkalten werden 200 ml Wasser zugetropft, dreimal mit je 100 ml Ethylacetat extrahiert, die vereinten organischen Phasen mit 50 ml Wasser gewaschen, über Natriumsulfat getrocknet und im Vakuum zur Trockne destilliert. Der Rückstand wird in 100 ml Chloroform gelöst, mit je 100 ml 1 N Natronlauge und 100 ml Wasser ausgeschüttelt, die organische Phase über Natriumsulfat getrocknet, zur Trockne destilliert und mittels Flüssigchromatographie gereinigt (Kieselgel 60 / 0,2-0,5 mm, Eluent Methylenchlorid/ Methanol = 9/1).
Ausbeute: 0,3 g (5 % der Theorie)
F. 46-49 °C
$^{13}C$-NMR (DMSO-$d_6$; 300 MHz): $\delta$ = 18,4 $CH_3S$ ; 51,7 $CH_2N$; 68,3 $CH_2O$; 72,7 $CH_2O$.

Die Synthese der Verbindung Beispiel 113 gelingt analog der Vorschrift nach Beispiel 112.

Tabelle 9: subst. 1-Benzyl-3-alkyloxy-5-methylthio-indazole

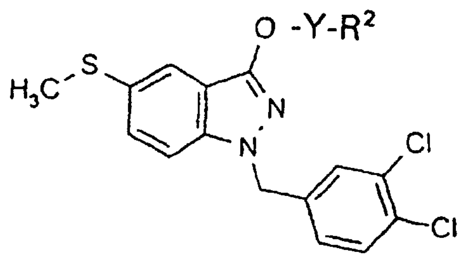

Formel XIV

| Beispiel | -Y-$R^2$ | Ausbeute (% d.Th.) | F. [°C] | $^{13}C$-NMR ($DMSO-d_6$) N-$CH_2$/ O-$CH_2$ |
|---|---|---|---|---|
| 113 | 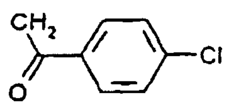 | 74 | 134-139 (EtOH) | 51,62/ 71,09 |

Beispiel 114

1-{1-(2,4-Dichlorbenzyl)-3-[2-(4-nitrophenoxy)ethoxy]-1H-indazol-5-yl}-3-naphthalen-1-ylharnstoff

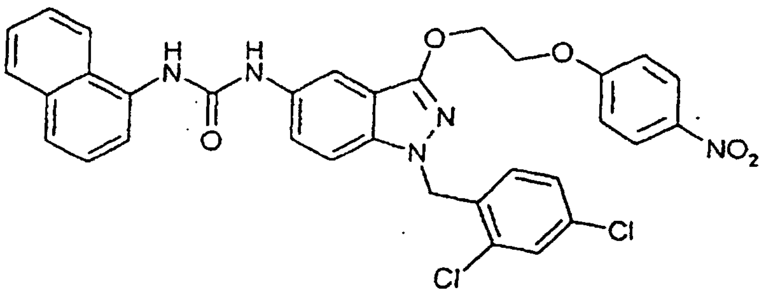

2,9 g (6,1 mmol) 1-[1-(2,4-Dichlorbenzyl)-3-hydroxy-1H-indazol-5-yl]-3-naphthalen-1-ylhamstoff werden in 70 ml DMSO gelöst und portionsweise mit 0,22 g (9 mmol) Natriumhydrid (95proz.) versetzt. Nach 10 Minuten Rühren wird eine Lösung von 1,5 g (6,1 mmol) 2-(4-Nitrophenoxy)-ethylbromid in 10 ml DMSO zugetropft und 3 Stunden bei 60°C gerührt. Nach dem Erkalten werden 400 ml Wasser zugegeben, 3 Stunden gerührt und dreimal mit 400 ml Ethylacetat extrahiert. Die vereinten organischen Phasen werden mit 100 ml Wasser gewaschen, über Natriumsulfat getrocknet, im Vakuum zur Trockne destilliert und der Rückstand aus Methanol umkristallisiert.
Ausbeute: 1,3 g (33,2 % der Theorie)
F. 179-183 °C

Tabelle 10: subst. 1-Benzyl-3-alkyloxy-indazol-5-amine

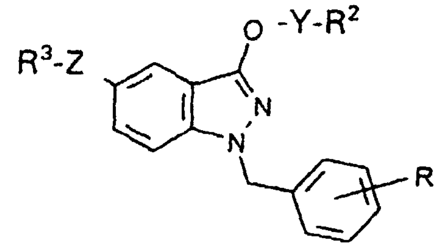

Formel XVI

R = 3,4-$Cl_2$

| Beispiel | $R^3$-Z- | -Y-$R^2$ | Ausbeute (% d.Th.) | F. [°C] | $^{13}$C-NMR (DMSO-$d_6$) N-$CH_2$/ O-$CH_2$ |
|---|---|---|---|---|---|
| 115 | 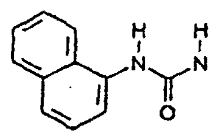 | 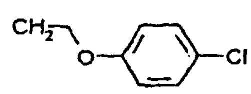 | 19 | 159-168 (MeOH) | 48,33 / 64,65; 65,35 |
| 116 | 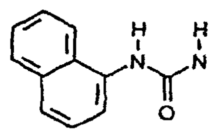 | 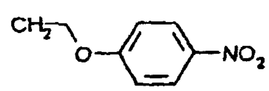 | 6 | 178-188 (MeOH) | 48,37 / 65,29; 65,48 |
| 117 | 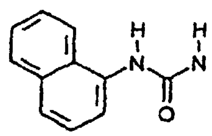 | 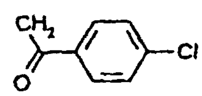 | 23 | 146-153 (MeOH) | 50,63 / 71,05 |
| 118 | 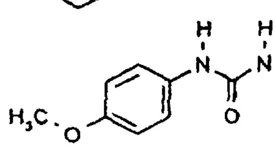 | 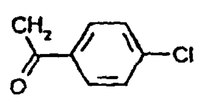 | 23 | 149-155 (MeOH) | 50,44 / 71,01 |
| 119 | 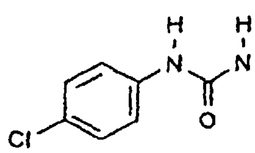 | 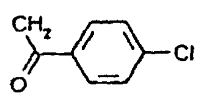 | 50 | 209-211 (BuOH) | 50,71 / 71,03 |

$^{13}$C-NMR (DMSO-$d_6$; 300 MHz): δ = 48,8 $CH_2N$; 67,1 $CH_2O$; 67,3 $CH_2O$.

Die Synthese der Verbindungen Beispiel 115 bis 123 gelingt analog der Vorschrift nach Beispiel 114.

Tabelle 10: subst. 1-Benzyl-3-alkyloxy-indazol-5-amine

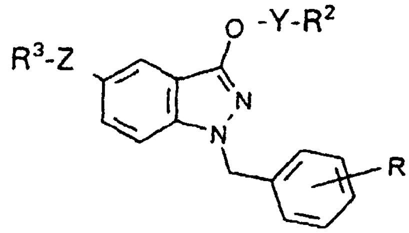

Formel XVI

R = 3,4-$Cl_2$

| Beispiel | $R^3$-Z- | -Y-$R^2$ | Ausbeute (% d.Th.) | F. [°C] | $^{13}$C-NMR ($DMSO-d_6$) N-$CH_2$/ O-$CH_2$ |
|---|---|---|---|---|---|
| 115 | 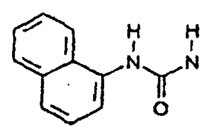 | 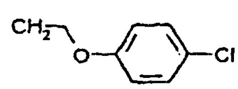 | 19 | 159-168 (MeOH) | 48,33 / 64,65; 65,35 |
| 116 | 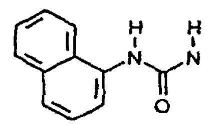 | 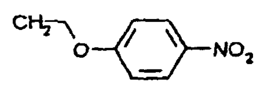 | 6 | 178-188 (MeOH) | 48,37 / 65,29; 65,48 |
| 117 | 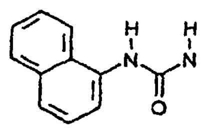 | 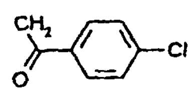 | 23 | 146-153 (MeOH) | 50,63 / 71,05 |
| 118 | 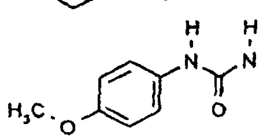 | 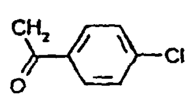 | 23 | 149-155 (MeOH) | 50,44 / 71,01 |
| 119 | 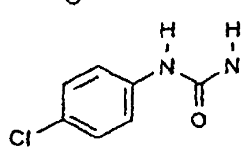 | 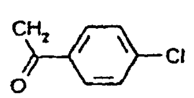 | 50 | 209-211 (BuOH) | 50,71 / 71,03 |

Formel XVI, R = 2,4-$Cl_2$

| Beispiel | $R^3$-Z- | -Y-$R^2$ | Ausbeute (% d.Th.) | F. [°C] | $^{13}$C-NMR ($DMSO-d_6$) N-$CH_2$/ O-$CH_2$ |
|---|---|---|---|---|---|
| 120 | 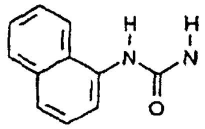 | 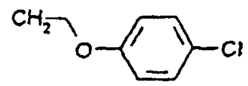 | 31 | 184-187 (Dioxan) | 49,13 / 66,93; 67,82 |
| 121 | 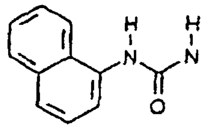 | 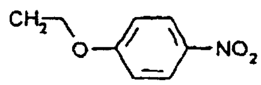 | 33 | 179-183 (MeOH) | 48,87 / 67,16; 67,33 |
| 122 | 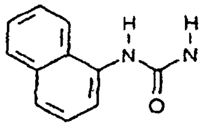 | 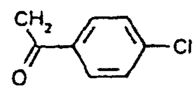 | 26 | 185-187 (BuOH) | 49,00 / 71,01 |
| 123 | 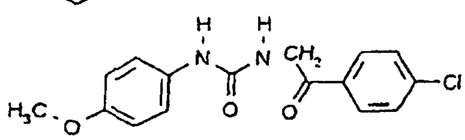 | | 39 | 192-196 (MeCN) | 48,98 / 70,98 |

Zur Bestimmung der Wirkung der erfindungsgemäßen Verbindungen auf antiasthmatische, antiallergische, entzündungshemmende und/oder immunmodulierende Eigenschaften wurden in vitro und in vivo Untersuchungen durchgeführt.
Die erfindungsgemäßen Verbindungen gemäß Formel **I** zeichnen sich überraschenderweise durch Immunophilin-Bindung aus und hemmen deren Peptidyl-Prolyl-cis-trans-Isomerase (PPlase)-Aktivität. Für das Eingangsscreening (10 µmol/l) wird die Inhibition des humanen Cyclophilin B im PPlase-Test bestimmt.

Assay zur Bestimmung der Peptidylprolylisomerase (PPlase)-Aktivität und Hemmung

Methode:

Die PPlase-Aktivität wird nach einem weltweit üblichen Enzym-Test geprüft: G. Fischer, H. Bang, C. Mech, Biomed. Biochim. Acta 43 1101-1111; G. Fischer, H. Bang, A. Schellenberger, Biochim. Biophys. Acta 791 (1984), 87-97; D. H. Rich et al., J. Med. Chem. 38 (1995), 4164-4170.
Die erfindungsgemäßen Verbindungen der allgemeinen Formel **I** werden zusammen mit 10 nmol Cyp B für 15 Minuten bei 4°C präinkubiert. Die Enzymreaktion wird nach Zugabe von Chymotrypsin und HEPES-Puffer mit dem Testpeptid Suc-Ala-Ala-Pro-Phe-Nan gestartet. Anschließend wird die Extinktionsänderung bei 390 nm verfolgt und ausgewertet. Die photometrisch ermittelte Extinktionsänderung resultiert aus zwei Teilreaktionen: a) die schnelle chymotryptische Spaltung des trans-Peptides; b) die nicht-enzymatische cis-trans-Isomerisierung, die durch Cyclophiline katalysiert ist. Die entsprechende PPlase-Aktivität der erfindungsgemäßen Verbindungen der allgemeinen Formel **I** sind in Tabelle 11 dargestellt:

Tabelle 11:

| Beispiel | Hemmung der PPlase-Aktivität in [%] bei 10 µM |
|---|---|
| 1 | 95 |

Tabelle 11: (fortgesetzt)

| Beispiel | Hemmung der PPlase-Aktivität in [%] bei 10 μM |
|---|---|
| 7 | 70 |
| 9 | 90 |
| 32 | 70 |
| 41 | 71 |
| 66 | 40 |
| 73 | 67 |
| 84 | 90 |
| 114 | 100 |
| 116 | 90 |
| 121 | 100 |

Hemmung der Spätphasen-Eosinophilie 24 h nach inhalativer Ovalbuminchallenge an aktiv sensibilisierten Meerschweinchen

Methode:

Die Hemmung der pulmonalen Eosinophilen-Infiltration durch die Substanzen wird in einem *in vivo* Test an aktiv gegen Ovalbumin (OVA) sensibilisierten männlichen Dunkin-Hartley Meerschweinchen (200-250 g) geprüft. Die Sensibilisierung erfolgt durch zwei intraperitoneale Injektionen einer Suspension von 20 μg OVA zusammen mit 20 mg Aluminiumhydroxid als Adjuvans in 0,5 ml physiologischer Kochsalzlösung pro Tier an zwei aufeinanderfolgenden Tagen. 14 Tage nach der zweiten Injektion werden die Tiere mit Mepyramin maleat (10 mg/kg i.p.) vorbehandelt, um sie vor dem anaphylaktischen Tod zu schützen. 30 Minuten später werden die Tiere in einer Plastikbox für 30 sec einem OVA-Aerosol ausgesetzt (0,5 mg/ml) das von einem mit Pressluft (19,6 kPa) getriebenen Vernebler erzeugt wird (Allergen-Challenge). Kontrolltiere werden mit physiologischer Kochsalzlösung vernebelt. 24 Stunden nach der Challenge werden die Tiere mit einer Überdosis Ethylurethan (1,5 g/kg Körpergewicht i.p.) narkotisiert und eine bronchoalveoläre Lavage (BAL) mit 2 x 5 ml physiologischer Kochsalzlösung durchgeführt. Die BAL-Flüssigkeit wird gesammelt, bei 300 rpm für 10 min zentrifugiert und anschließend das Zellpellet in 1 ml physiologischer Kochsalzlösung resuspendiert. Die Eosinophilen werden unter Verwendung des Becton-Dickinson Test Kit (N. 5877) für Eosinophile gefärbt und in einer Neubauerkammer gezählt. Bei jedem Test werden 2 Kontrollgruppen (Vernebelung mit physiologischer Kochsalzlösung und Vernebelung mit OVA-Lösung) mitgeführt.

Die prozentuale Hemmung der Eosinophilie der mit Substanz behandelten Versuchsgruppe wird nach folgender Formel berechnet:

$$(A - C) - (B - C)/ (A - C) \times \% \text{ Hemmung}$$

Die Testsubstanzen werden intraperitoneal oder oral als Suspension in 10 % Polyethylenglycol 300 und 0,5 %iger 5-Hydroxyethylcellulose 2 Stunden vor der Allergen-Challenge appliziert. Die Kontrollgruppen werden entsprechend der Applikationsform der Testsubstanz mit dem Vehicel behandelt. Die Anzahl der Tiere pro Kontroll- und Versuchsgruppe beträgt 3-10. Die Ergebnisse sind in der nachfolgenden Tabelle aufgeführt:

Tabelle 12:

| Beispiel | Dosis [mg/kg] | Applikation | Eosinophile Mio/Tier $\bar{x} \pm s$ | | | Hemmung [%] |
|---|---|---|---|---|---|---|
| | | | A | B | C | |
| 1 | 10 | i.p. - 2h | 2,11 ± 1,05 | 1,23 ± 0,38 | 0,67 ± 0,23 | 61 |
| | 30 | p.o. - 2h | 3,49 ± 1,47 | 1,75 ± 1,86 | 0,83 ± 0,30 | 65 |
| 8 | 10 | i.p. - 2h | 2,46 ± 1,08 | 1,84 ± 0,94 | 0,97 ± 0,47 | 41 |

Tabelle 12: (fortgesetzt)

| Beispiel | Dosis [mg/kg] | Applikation | Eosinophile Mio/Tier $\bar{x} \pm s$ | | | Hemmung [%] |
|---|---|---|---|---|---|---|
| | | | A | B | C | |
| 32 | 10 | i.p. - 2h | 1,93 ± 0,75 | 0,86 ± 0,49 | 0,66 ± 0,12 | 85 |
| 91 | 10 | i.p. - 2h | 2,89 ± 1,66 | 1,16 ± 0,65 | 0,47 ± 0,24 | 71 |
| 99 | 10 | i.p. - 2h | 1,93 ± 0,75 | 1,35 ± 0,67 | 0,66 ± 0,12 | 46 |
| | 30 | p.o. - 2h | 1,81 ± 0,23 | 1,33 ± 0,23 | 0,33 ± 0,08 | 33 |
| 107 | 10 | i.p. - 2h | 2,46 ± 1,08 | 1,44 ± 0,92 | 0,97 ± 0,47 | 68 |
| 114 | 10 | i.p. - 2h | 1,93 ± 0,75 | 1,19 ± 0,43 | 0,66 ± 0,12 | 58 |

A = Eosinophile in der Kontrollgruppe mit OVA-Challenge und Vehicel
B = Eosinophile in der mit Substanz behandelten Gruppe mit OVA-Challenge
C = Eosinophile in der Kontrollgruppe mit 0,9 %iger NaCl-Challenge und Vehicel
$\bar{x}$ = Mittelwert s = Standardabweichung

Die erfindungsgemäßen Verbindungen sind somit besonders geeignet für die Herstellung von Arzneimitteln zur Behandlung von Erkrankungen, die mit der Unterdrückung immunologischer Vorgänge verbunden sind.

## Patentansprüche

**1.** 1,5- und 3-O- substituierte 1H-Indazole der allgemeinen Formel I

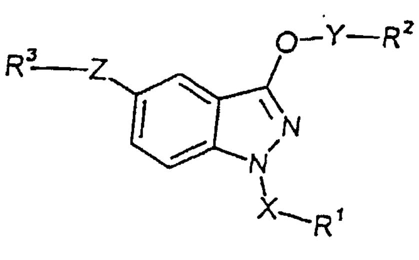

Formel I

worin X, Y, Z, $R^1$, $R^2$ und $R^3$ folgende Bedeutung haben:

X kann $-SO_2-$, $-(CH_2)_p-$, $-(CH_2)_p-O-$ mit p = 1...4 sein,

Y kann $-(CH_2)_p-$, $-(CH_2)_p-O-$, $-(CH_2)_p-(C=O)-$, $-(CH_2)_p-(C=O)-NH-$, $-(CH_2)_p-CHOH-$, $-(CH_2)_p-CH=CH-$, mit p = 1...4 sein,

Z kann -O-, -S-, -NH-(C = O)-NH- sein,

$R^1$ kann folgende Bedeutung haben:
Naphthyl, Chinolyl oder Phenyl, wobei die Carbocyclen substituiert sein können mit:

$-C_{1...6}$-Alkyl, $-O-C_{1...6}$-Alkyl, -F, -Cl, $-NO_2$, $-NH_2$, $-(CO)C_{1...6}$-Alkyl, $-S-C_{6...14}$-Aryl,

$R^1$ kann weiter H sein, mit der Maßgabe, dass $R^1$ nicht H ist, wenn X = $CH_2$;

$R^2$ kann folgende Bedeutung haben:
Phenyl, oder mono- oder bicyclische gesättigte oder ein- oder mehrfach ungesättigte Heterocyclen mit 5...15 Ringgliedern und 1...6 Heteroatomen;
wobei das Phenyl bzw. die Heterocyclen einfach oder mehrfach substituiert sein können mit:
$-C_{1...6}$-Alkyl, $-O-C_{1...6}$-Alkyl, monocyclische gesättigte Carbocyclen mit 3...14 Ringgliedern, -F, -Cl, -Br, $-NO_2$, $-NH_2$, -CN, $-(CO)C_{1...6}$-Alkyl, Benzyloxy, $-CF_3$,

$R^3$ kann folgende Bedeutung haben:
Methyl, Napthyl oder Phenyl, wobei das Phenyl einfach oder mehrfach substituiert sein kann mit:
$-O-C_{1...6}$-Alkyl, -Cl.

**2.** 1,5- und 3-O-substituierte 1H-Indazole, ausgewählt aus 3-[2-(2-Brom-4,6-difluorphenoxy)ethoxy]- 5-methoxy-1-(toluol-4-sulfonyl)-1H-indazol, 5-Methoxy-3-[2-(phenoxy)ethoxy]-1-(toluol-4-sulfonyl)-1H-indazol, 5-Methoxy-3-[3-(phenoxy)propoxy]-1-(toluol-4-sulfonyl)-1H-indazol3-[4-(Butoxy)propoxy]-5-methoxy-1-(toluol-4-sulfonyl)-1H-indazol, 3-[2-(2-Bromphenoxy)ethoxy]-5-methoxy-1-(toluol-4-sulfonyl)-1H-indazol, 3-[2-(4-Chlorphenoxy)ethoxy]-5-methoxy-1-(toluol-4-sulfonyl)-1H-indazol, 3-[2-(4-Chlorphenoxy)ethoxy]-5-methoxy-1-(4-chlorbenzensulfonyl)-1Hindazol,3-[2-(4-Chlorphenoxy)ethoxy]-5-methoxy-1-(4-fluorbenzensulfonyl)-1H-indazol,3-[2-(4-Chlorphenoxy)ethoxy]-5-methoxy-1-(4-methoxybenzensulfonyl)-1H-indazol, 3-[2-(4-Chlorphenoxy)ethoxy]-5-methoxy-1-(4-trifluormethoxybenzensulfonyl)-1H-indazol, 3-[2-(2,6-Dichlorphenoxy)ethoxy]-5-methoxy-1-(toluol-4-sulfonyl)-1H-indazol, 3-[2-(2,4-Difluorphenoxy)ethoxy]-5-methoxy-1-(toluol-4-sulfonyl)-1H-indazol, 3-[2-(2,6-Difluorphenoxy)ethoxy]-5-methoxy-1-(toluol-4-sulfonyl)-1H-indazol, 5-Methoxy-1-(toluol-4-sulfonyl)-3-[2-(2,4,6-trifluorphenoxy)ethoxy]-1H-indazol,3-[2-(2-Brom-4,6-Difluorphenoxy)ethoxy]-5-methoxy-1-(4-methoxybenzensulfonyl)-1H-indazol, 3-[2-(2-Brom-4,6-Difluorphenoxy)ethoxy]-5-methoxy-1-(4-chlorbenzensulfonyl)-1H-indazol,3-[2-(2,6-Dibrom-4-nitrophenoxy)ethoxy]-5-methoxy-1-(toluol-4-sulfonyl)-1H-indazol,3-[2-(2,6-Dibrom-4-nitrophenoxy)ethoxy]-5-methoxy-1-(4-methoxybenzensulfonyl)-1H-indazol, 5-Methoxy-3-[2-(4-nitrophenoxy)ethoxy]- 1-(toluol-4-sulfonyl)-1H-indazol, 1-(4-Chlorbenzensulfonyl)-5-methoxy-3-[2-(4-nitrophenoxy)ethoxy]-1H-indazol, 5-Methoxy-1-(4-methoxybenzensulfonyl)-3-[2-(4-nitrophenoxy)ethoxy]-1H-indazol, 5-Methoxy-3-[2-(4-nitrophenoxy)ethoxyl-1-(thiophen-2-sulfonyl)-1H-indazol, 3-[2-(4-Cyanophenoxy)ethoxy]-5-methoxy-1-(toluol-4-sulfonyl)-1H-indazol, 3-[2-(4-Carboxamidophenoxy)ethoxy]-5-methoxy-1-(toluol-4-sulfonyl)-1Hindazol, 5-Methoxy-1-(toluol-4-sulfonyl)-3-[5-(3-trifluormethylphenyl)-1,2,4-oxadiazol-3-ylmethoxy]-1H-indazol, 3-(4-Benzyloxy-benzyloxy)-5-methoxy-1-(toluol-4-sulfonyl)-1H-indazol, 3-(5-Acetyl-2-methoxybenzyloxy)-5-methoxy-1-(toluol-4-sulfonyl)-1H-indazol, 3-(2-Chlor-6-fluorbenzyloxy)-5-methoxy-1-(toluol-4-sulfonyl)-1H-indazol, 1-(4-Chlorbenzensulfonyl)-3-(2-chlor-6-fluorbenzyloxy)-5-methoxy-1Hindazol, 1-(4-Fluorbenzensulfonyl)-5-methoxy-3(3-trifluormethylbenzyloxy)-1H-indazol, 3-(2-Fluorbenzyloxy)-5-methoxy-1-(toluol-4-sulfonyl)-1H-indazol, 1-(4-Fluorbenzensulfonyl)-3-(2-fluorbenzyloxy)-5-methoxy-1H-indazol,1-(4-Fluorbenzensulfonyl)-3-(4-fluorbenzyloxy)-5-methoxy-1H-indazol,3-(4-Chlor-2-nitrobenzyloxy)-5-methoxy-1-(toluol-4-sulfonyl)-1H-indazol, 3-(6-Chlor-benzo[1,3]dioxo-5-ylmethoxy)-5-methoxy- 1-(toluol-4-sulfonyl)-1H-indazol, 3-(6-Fluor-4Hbenzo[1,3]dioxin-8-ylmethoxy)-5-methoxy-1-(toluol-4-sulfonyl)-1Hindazol, 5-Methoxy-3-(6-nitro-4H-benzo[1,3]dioxin-8-ylmethoxy)-1-(toluol-4-sulfonyl)-1H-indazol, 5-Methoxy-3-(6-nitro-4Hbenzo[1,3]dioxin-8-ylmethoxy)-1-(4-fluorbenzensulfonyl)-1H-indazol3-(3,5-Dimethyl-isoxazol-4-ylmethoxy)-5-methoxy-1-(toluol-4-sulfonyl)-1H-indazol, 3-(3,5-Dimethyl-isoxazol-4-ylmethoxy)-5-methoxy-1-(4-chlorbenzensulfonyl)-1H-indazol,5-Methoxy-3-[5-(2-methoxyphenyl)[1,2,4]oxadiazol-3-ylmethyl]-1-( toluol-4-sulfonyl)-1Hindazol, 5-Methoxy-3-[5-(4-tert.-butylphenyl)[1,2,4]oxadiazol-3-ylmethyl]- 1-( toluol-4-sulfonyl)-1H-indazol, 3-[2-(4-Chlorphenyl)-thiazol-4-ylmethyl]- 5-methoxy-1-(toluol-4-sulfonyl)-1H-indazol, 6-{2-[5-Methoxy-1-(toluol-4-sulfonyl)-1H-indazol-3-yloxy]acetyl}-3,4-dihydro-1H-chinolin-2-on, 3-[2-(4-Chlorphenyl)-2-oxo-ethoxy]-5-methoxy-1-(toluol-4-sulfonyl)-1H-indazol, 3-[2-(4-Chlorphenyl)-2-oxo-ethoxy]-5-methoxy-1-(4-methoxybenzensulfonyl)-1H-indazol, 3-[2-(4-Chlorphenyl)-2-oxo-ethoxy]-5-methoxy-1 -(4-chlorbenzensulfonyl)-1H-indazol, 3-[2-(3,4-Dichlorphenyl)-2-oxo-ethoxy]-5-methoxy-1-(toluol-4-sulfonyl)-1Hindazol, 3-[2-(3,4-Dichlorphenyl)-2-oxo-ethoxy]-5-methoxy-1-(4-chlorbenzensulfonyl)-1H-indazol, 3-[2-(3,4-Dichlorphenyl)-2-oxoethoxy]-5-methoxy-1-(4-acetylamino-benzensulfonyl)-1H-indazol,3-[2-(3,4-Dichlorphenyl)-2-oxo-ethoxy]-5-methoxy-1-(naphthalen-1-sulfonyl)-1H-indazol, 3-[2-(3,4-Dichlorphenyl)-2-oxo-ethoxy]-5-methoxy-1-(chinolin-8-sulfonyl)-1H-indazol, 3-[2-(4-Diphenyl)-2-oxo-ethoxy]-5-methoxy-1-(toluol-4-sulfonyl)-1H-indazol, 3-[2-(4-Diphenyl)-2-oxoethoxyl-5-methoxy-1-(4-chlorbenzensulfonyl)-1H-indazol, 3-[2-(4-Diphenyl)-2-oxo-ethoxy]-5-methoxy-1-(4-methoxybenzensulfonyl)-1Hindazol, 3-[2-(4-Diphenyl)-2-oxo-ethoxyl-5-methoxy-1-(4-acetylaminobenzensulfonyl)-1H-indazol, N-(2,4-Difluorphenyl)-2-[5-methoxy-1-(toluol-4-sulfonyl)-1H-indazol-3-yloxyl-acetamid, 5-Methoxy-3-(3-phenylallyloxy)-1-(toluol-4-sulfonyl)-1H-indazol, 1-(2,4-Dichlorphenyl)-2-[5-methoxy-1-(toluol-4-sulfonyl)-1H-indazol-3-yloxy]-ethanol, 1-(4-Methoxyphenyl)-4-[5-methoxy-1-(toluol-4-sulfonyl)-1H-indazol-3-yloxy]-butan-1-on, 1-(4-Methoxyphenyl)-4-[1-(4-fluorbenzensulfonyl)-5-methoxy-1H-indazol-3-yloxy]-butan-1-on, 1-(4-Fluorphenyl)-4-[5-methoxy-1-(toluol-4-sulfonyl)-1H-indazol-3-yloxyl-butan-1-on, N-(4-Methoxyphenyl)-2-[5-methoxy-1-(toluol-4-sulfonyl)-1H-indazol-3-yloxy]-acetamid N-(2,4-Dichlorphenyl)-2-[5-methoxy-1-(toluol-4-sulfonyl)-1Hindazol-3-yloxy]-acetamid N-Benzyl-2-[5-methoxy-1-(toluol-4-sulfonyl)-1H-indazol-3-yloxy]-acetamid, 3-{2-[5-Methoxy-1-(toluol-4-sulfonyl)-1Hindazol-3-yloxy]ethyl}-1H-chinazolin-2,4-dion, 3-{2-[5-Methoxy-1-(4-methoxybenzensulfonyl)-1H-indazol-3-yloxy]ethyl}-1H-chinazolin-2,4-dion, 3-(6-Chlorbenzo [1,3] dioxol-5-ylmethoxy)-5-methoxy-1-(3-nitrobenzyl)-1H-indazol3-[2-(4-Chlorphenoxy)ethoxy]-5-methoxy-1-(3-nitrobenzyl)-1H-indazol,3-[2-(4-Nitrophenoxy)ethoxy]-5-methoxy-1-(3-nitrobenzyl)-1H-indazol, 3-[2-(4-Carboxaminophenoxy)ethoxy]-5-methoxy-1-(3-nitrobenzyl)-1H-indazol,3-[2-(2-Brom-4,6-difluorphenoxy)ethoxy]-5-methoxy-1 -(3-nitrobenzyl)-1H-indazol, 3-[2-(2,6-Dibrom-4-nitrophenoxy)ethoxy]-5-methoxy-1-(3-nitrobenzyl)-1Hindazol, 5-Methoxy-1-(3-nitrobenzyl)-3-(pyridin-4-ylmethoxy)-1H-indazol Hydrochlorid, 3-(2,6-Dichlorbenzyloxy)-5-methoxy-1-(3-nitrobenzyl)-1H indazol,3-[2-(4-Chlorphenyl)acetoxy]-5-methoxy-1-(3-nitrobenzyl)-1Hindazol, 3-[3-(6-Chlorbenzo [1,3] dioxol-5-ylmethoxy)-5-methoxyindazol-1-ylmethyl]-phenylamin, 4-{2-[1-(3-Aminobenzyl)-5-methoxy-1H-indazol-3-yloxy]-ethoxy}-phenylamin Dihydrochlorid, 4-{2-[1-(3-Aminobenzyl)-5-methoxy-1H-indazol-3-yloxy]-ethoxy}-benzamid, 3-[3-(4-Chlorphenylcarboxymethyloxy)-5-methoxy-indazol-1-ylmethyl]-phenylamin Hydrochlorid, 1-(4-Fluorbenzyl)-5-methoxy-3-[2-(4-nitrophenoxy)ethoxy]-1H-indazol, 3-[2-(4-Chlorphenoxy)ethoxy]-1-(4-fluorbenzyl)-5-methoxy-1H-indazol, 3-[2-(4-Aminophenoxy)ethoxy]-1-(4-fluorbenzyl)-5-methoxy-1H-indazol, 3-[2-(2-Brom-4,6-difluorphenoxy)ethoxy]-1-(4-fluorbenzyl)-5-methoxy-1H-indazol, 3-(3,5-Dimethylisoxazol-4-ylmethoxy)-1-(4-fluorbenzyl)-5-methoxy-1H-indazol, 3-(2,3,6-Trifluorbenzyloxy)-1-(4-fluorbenzyl)-5-methoxy-1H-indazol, 3-(6-Chlorbenzo[1,3]dioxol-5-ylmethoxy)-1-(4-fluorbenzyl)-5-methoxy-1Hindazol,3-(4-Chlorphenylcarboxymethyloxy)-1-(4-fluorbenzyl)-5-methoxy-1H-indazol, 3-[2-(2-Brom-4,6-difluorphenoxy)ethoxy]-5-methoxy-1-[2-(4-nitrophenoxy)ethyl]-1H-indazol, 3-[2-(4-Chlorphenoxy)ethoxy]-5-methoxy-1-[2-(4-nitrophenoxy)ethyl]-1Hindazol, 3-[2-(2,6-Dibrom-4-nitrophenoxy)ethoxy]-5-methoxy-1-[2-(4-nitrophenoxy)ethyl]-1H-indazol,3-(4-Chlorphenylcarboxymethyloxy)-5-methoxy-1-[2-(4-nitrophenoxy)ethyl]-1H-indazol, 3-(3,4-Dichlorphenylcarboxymethyloxy)-5-methoxy-1-[2-(4-nitrophenoxy)ethyl]-1H-indazol,3-(4-Diphenylcarboxymethyloxy)-5-methoxy-1-[2-(4-nitrophenoxy)ethyl]-1H-indazol, 6-(2-{5-Methoxy-1-[2-(4-nitrophenoxy)ethyl]-1H-indazol-3-yloxy}acetyl]-3,4-dihydro-1Hchinolin-2-on, N-(2,4-Difluorphenyl)-2-{5-methoxy-1-[2-(4-nitrophenoxy)ethyl]-1H-indazol-3-yloxy}acetamid, 3-(3-Acetyl-6-methoxybenzyloxy)-5-methoxy-1-[2-(4-nitrophenoxy)ethyl]-1H-indazol, 3-(3-Trifluormethylbenzyloxy)-5-methoxy-1-[2-(4-nitrophenoxy)ethyl]-1H-indazol, 3-(2-Fluormethylbenzyloxy)-5-methoxy-1-[2-(4-nitrophenoxy)ethyl]-1H-indazol, 3-(6-Fluor-4H-benzo[1,3]dioxin-8-ylmethoxy)-5-methoxy-1-[2-(4-nitrophenoxy)ethyl]-1H-indazol,3-[2-(4-Chlorphenoxy)ethoxy]-1-[2-(4-chlorphenoxy)ethyl]-5-methoxy-1H-indazol,3-(4-Chlorphenoxyalkyloxy)-5-amino-1-(toluol-4-sulfonyl)-1Hindazol, 3-(2,6-Dibrom-4-nitrophenoxyalkyloxy)-5-amino-1-(toluol-4-sulfonyl)-1H-indazol und 3-(4-Chlorphenylcarboxymethyloxy)-5-amino-1-(toluol-4-sulfonyl)-1H-indazol.

**3.** Verbindungen nach einem der vorhergehenden Ansprüche, ausgewählt aus

3-[2-(2-Brom-4,6-difluorphenoxy)ethoxy]-5-methoxy-1-(toluol-4-sulfonyl)-1H-indazol,
5-Methoxy-1-(toluol-4-sulfonyl)-3-[5-(3-trifluormethylphenyl)-1,2,4-oxadiazol-3-ylmethoxyl-1H-indazol,
6-{2-[5-Methoxy-1-(toluol-4-sulfonyl)-1 H-indazol-3-yloxy]acetyl}-3,4-dihydro-1H-chinolin-2-on,
N-(2,4-Difluorphenyl)-2-[5-methoxy-1-(toluol-4-sulfonyl)-1H-indazol-3-yloxy]-acetamid,

3-(6 Chlorbenzo [1,3]dioxol-5-ylmethoxy)-5-methoxy-1-(3-nitrobenzyl) -1H-indazol,
3-[3-(4-Fluorphenyl)-propoxy]-5-nitro-1-(3-nitrobenzyl)-1H-indazol,
3-[3-(6-Chlorbenzo [1,3] dioxol-5-ylmethoxy)-5-methoxy-indazol -1-ylmethyl]-phenylamin,
1-(4-Fluorbenzyl)-5-methoxy-3-[2-(4-nitrophenoxy)ethoxy]-1H-indazol,
3-[2-(2-Brom-4,6-difluorphenoxy)ethoxy]-5-methoxy-1-[2-(4-nitrophenoxy)ethyl]-1H-indazol,
3-[2-(2-Brom-4,6-difluorphenoxy)ethoxy]-1-[3,4-dichlorbenzyl]-5-methylthio-1H-indazol und
1-1-{1-(2,4-Dichlorbenzyl)-3-[2-(4-nitrophenoxy)ethoxy]-1H -indazol-5-yl} -3-naphthalen-1-ylharnstoff.

**4.** Verbindung nach einem der vorhergehenden Ansprüche, wobei die Verbindung 3-[2-(2-Brom-4,6-difluorphenoxy) ethoxy]5-methoxy-1-(toluol-4-sulfonyl)-1H-indazol ist.

**5.** Verbindung nach einem der vorhergehenden Ansprüche, wobei die Verbindung 3-[2-(4-Chlorphenoxy)ethoxy]-5-methoxy-1-1(4-fluorbenzensulfonyl)-1H-indazol ist.

**6.** Physiologisch verträgliche Salze der Verbindungen gemäß einem der Ansprüche 1 bis 5, **gekennzeichnet durch**

Neutralisation der Basen mit anorganischen oder organischen Säuren bzw. **durch** Neutralisation der Säuren mit anorganischen oder organischen Basen bzw. **durch** Quaternierung tertiärer Amine zu quaternären Ammoniumsalzen.

**7.** Verwendung der Verbindungen nach einem der Ansprüche 1 bis 5 odeer deren Salzen gemäß Anspruch 6 als therapeutische Wirkstoffe zur Herstellung von Arzneimitteln zur Behandlung von durch die PPlase vermittelten Erkrankungen.

**8.** Verwendung der Verbindungen nach einem der Ansprüche 1 bis 5 oder deren Salzen gemäß Anspruch 6 als therapeutische Wirkstoffe zur Herstellung von Arzneimitteln zur Behandlung von Erkrankungen, die mit der Unterdrückung immunologischer Vorgänge verbunden sind.

**9.** Verwendung der Verbindungen nach einem der Ansprüche 1 bis 5 oder deren Salze gemäß Anspruch 6 als therapeutische Wirkstoffe zur Herstellung von Arzneimitteln zur Behandlung von Psoriasis.

**10.** Verwendung der Verbindungen nach einem der Ansprüche 1 bis 5 oder deren Salze gemäß Anspruch 6 als therapeutische Wirkstoffe zur Herstellung von Arzneimittel zur Behandlung'von Asthma bronchiale, allergischer Rhinitis, allergischer Konjugtivitis, atopischer Dermatitis, Ekzemen, allergischer Angiitis, durch Eosinophile vermittelte Entzündungen wie eosinophile Fasciitis, eosinophile Pneumonie und PIE-Syndrom, Autoimmunerkrankungen wie rheumatoide Arthritis, rheumatoide Spondylitis, Lupus erythematosus, Multiple Sklerose, Glomerulonephritis und Uveitis, Insulin-abhängiger Diabetes mellitus und Sepsis.

**11.** Verfahren zur Herstellung einer Verbindung gemäß einem der Ansprüche 1 bis 6, **dadurch gekennzeichnet, dass**

a) für X = $-SO_2-$ entsprechend Schema 1 verfahren wird, indem Sulfonsäure-1H-indazol-3-ylester II

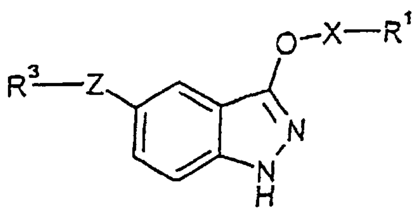

**Formel II**

in Gegenwart einer Base und gegebenenfalls in Gegenwart eines Verdünnungsmittels zu Verbindungen der allgemeinen Formel III

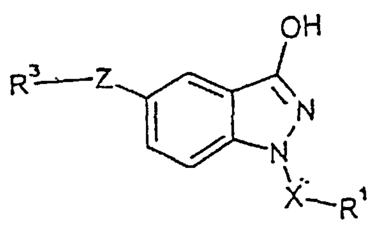

**Formel III**

umgesetzt werden, wobei $R^1$, $R^3$, X und Z die im Anspruch 1 bzw. oben genannte Bedeutung besitzen, und Sulfonsäure-1H-indazol-3-ylester **II** oder 1-Sulfonyl-indazole **III** gegebenenfalls in Gegenwart einer Base, und gegebenenfalls in Gegenwart eines Verdünnungsmittels, mit Verbindungen der allgemeinen Formel Hal-Y-$R^2$, wobei $R^1$, $R^2$, $R^3$, X, Y und Z die im Anspruch 1 bzw. oben genannte Bedeutung besitzen und Hal ein Halogenatom F, Cl, Br oder Jod bedeutet, zu Verbindungen der allgemeinen Formel **I** umgesetzt werden, wobei $R^1$, $R^2$, $R^3$, X, Y und Z die im Anspruch 1 bzw. oben genannte Bedeutung besitzen,

b) für X = $-(CH_2)_p-$, $-(CH_2)_p-O-$ mit p = 1...4 entsprechend Schema 2 verfahren wird, indem

Verbindungen der allgemeinen Formel **III** gegebenenfalls in Gegenwart einer Base, und gegebenenfalls in Gegenwart eines Verdünnungsmittels, mit Verbindungen der allgemeinen Formel Hal-Y-$R^2$, wobei $R^1$, $R^2$, $R^3$, X, Y und Z die im Anspruch 1 bzw. oben genannte Bedeutung besitzen und Hal ein Halogenatom F, Cl, Br oder Jod bedeutet, zu Verbindungen der allgemeinen Formel I umgesetzt werden, wobei $R^1$, $R^2$, $R^3$, X, Y und Z die im Anspruch 1 bzw. oben genannte Bedeutung besitzen,

wobei Formel III auch als tautomere Fom Formel IV

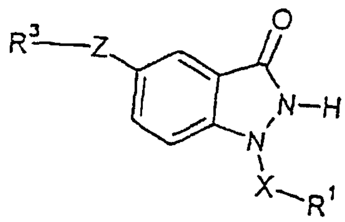

**Formel IV**

entsprechend Schema 3 vorliegen kann.

**12.** Verfahren nach Anspruch 11, worin als Base Natriumhydrid oder/und als Verdünnungsmittel Dimethylsulfoxid eingesetzt wird.

**13.** Pharmazeutisches Mittel, **dadurch gekennzeichnet, dass** es als wirksamen Bestandteil mindestens eine Verbindung nach einem der Ansprüche 1 bis 6 und physiologisch verträgliche Träger- und/oder Verdünnungs- bzw. Hilfsstoffe enthält.

**14.** Pharmazeutische Zubereitungen, **dadurch gekennzeichnet, dass** sie als wirksamen Bestandteil mindestens eine Verbindung nach einem der Ansprüche 1 bis 6 und einen geeigneten Trägerstoff enthalten.

**15.** Pharmazeutische Präparate nach einem der Ansprüche 13 oder 14, **dadurch gekennzeichnet, dass** sie in Form von Tabletten, Dragees, Kapseln, Aerosolen, Pulverformulierungen, Pflastern, Lösungen, Ampullen oder Suppositorien verabreicht werden.

**16.** Verwendung einer Verbindung nach einem der Ansprüche 1 bis 6 und/oder von pharmazeutischen Zubereitungen nach einem der Ansprüche 13 oder 14 zur Herstellung eines Mittels mit antiasthmatischen, antiallergischen, entzündungshemmenden und/oder immunmodulierenden Wirkungen allein oder in Kombination untereinander oder in Kombination mit Trägerstoffen.

## Claims

**1.** 1,5- and 3-O-substituted 1H-indazoles of the general formula **I**

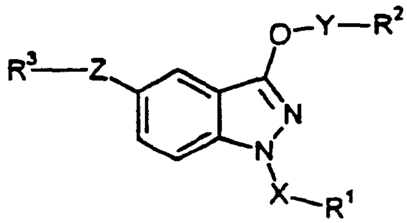

Formula I

in which X, Y, Z, $R^1$, $R^2$ and $R^3$ have the following meaning:

X can be $-SO_2-$, $-(CH_2)_p-$, $-(CH_2)_p-O-$, where p = 1...4,

Y can be $-(CH_2)_p-$, $-(CH_2)_p-O-$, $-(CH_2)_p-(C{=}O)-$, $-(CH_2)_p-(C{=}O)-NH-$, $-(CH_2)_p-CHOH-$, $-(CH_2)_p-CH{=}CH-$, where p = 1...4,

Z can be -O-, -S-, -NH-(C=O)-NH-,

$R^1$ can have the following meaning:
naphthyl, quinolyl or phenyl where the carbocycles can be substituted by:
$-C_{1...6}$-alkyl, $-O-C_{1...6}$-alkyl, -F, -Cl, $-NO_2$, $-NH_2$, $-(CO)C_{1...6}$-alkyl, $-S-C_{6...14}$-aryl,

$R^1$ can furthermore be H, with the proviso that $R^1$ is not H if X = $CH_2$,

$R^2$ can have the following meaning:
phenyl, or mono- or bicyclic saturated or mono- or polyunsaturated heterocycles having 5...15 ring members and 1...6 heteroatoms;
where the phenyl or the heterocycles, respectively, can be substituted one or more times by:
$-C_{1...6}$-alkyl, $-O-C_{1...6}$-alkyl, monocyclic saturated carbocycles having 3...14 ring members, -F, -Cl, -Br, $-NO_2$, $-NH_2$, -CN, $-(CO)C_{1...6}$-alkyl, benzyloxy, $-CF_3$,

$R^3$ can have the following meaning:
methyl, naphthyl or phenyl, where the phenyl can be substituted one or more times by:
$-O-C_{1...6}$-alkyl, -Cl.

**2.** 1,5- and 3-O-substituted 1H-indazoles, selected from 3-[2-(2-bromo-4,6-difluorophenoxy)ethoxy]-5-methoxy-1-(toluene-4-sulphonyl)-1H-indazole, 5-methoxy-3-[2-(phenoxy)ethoxy]-1-(toluene-4-sulphonyl)-1H-indazole, 5-methoxy-3-[3-(phenoxy)propoxy]-1-(toluene-4-sulphonyl)-1H-indazole, 3-[4-(butoxy)propoxy]-5-methoxy-1-(toluene-4-sulphonyl)-1H-indazole, 3-[2-(2-bromophenoxy)ethoxy]-5-methoxy-1-(toluene-4-sulphonyl)-1H-indazole, 3-[2-(4-chloro-phenoxy)-ethoxy]-5-methoxy-1-(toluene-4-sulphonyl)-1H-indazole, 3-[2-(4-chlorophenoxy)ethoxy]-5-methoxy-1-(4-chlorobenzenesulphonyl)-1H-indazole, 3-[2-(4-chlorophenoxy)ethoxy]-5-methoxy-1-(4-fluorobenzenesulphonyl)-1H-indazole, 3-[2-(4-chlorophenoxy)ethoxy]-5-methoxy-1-(4-methoxybenzenesulphonyl)-1H-indazole, 3-[2-(4-chlorophenoxy)ethoxy]-5-methoxy-1-(4-trifluoromethoxybenzenesulphonyl)-1H-indazole, 3-[2-(2,6-dichlorophenoxy)ethoxy]-5-methoxy-1-(toluene-4-sulphonyl)-1H-indazole, 3-[2-(2,4-difluorophenoxy)ethoxy]-5-methoxy-1-(toluene-4-sulphonyl)-1H-indazole, 3-[2-(2,6-difluorophenoxy)ethoxy]-5-methoxy-1-(toluene-4-sulphonyl)-1H-indazole, 5-methoxy-1-(toluene-4-sulphonyl)-3-[2-(2,4,6,-trifluorophenoxy)ethoxy]-1H-indazole, 3-[2-(2-bromo-4,6-difluoro-phenoxy)ethoxy]-5-methoxy-1-(4-methoxybenene-sulphonyl)-1H-indazole,3-(2-(2-bromo-4,6,difluorophenoxy)ethoxy]-5-methoxy-1-(4-chlorobenzenesulphonyl)-1H-indazole, 3-[2-(2,6-dibromo-4-nitrophenoxy)ethoxy]-5-methoxy-1-(toluene-4-sulphonyl)-1H-indazole, 3-[2-(2,6-dibromo-4-nitrophenoxy)ethoxy]-5-methoxy-1-(4-methoxybenzenesuflonyl)-1H-indazole, 5-methoxy-3-[2-(4-nitrophenoxy)ethoxy]-1-(toluene-4-sulphonyl)-1H-indazole, 1-(4-chlorobenzene-sulphonyl)-5-methoxy-3-[2-(4-nitrophenoxy)ethoxy]-1H-indzole, 5-methoxy-1-(4-methoxybenzene-sulphonyl)-3-[2-(4-nitrophenoxy)ethoxy)-1H-indazole, 5-methoxy-3-[2-(4-nitrophenoxy)ethoxy]-1-thiophene-2-sulphonyl)-1H-indazole, 3-[2-(4-cyanophenoxy)ethoxy]-5-methoxy-1-(toluene-4-sulphonyl)-1H-indazole, 3-[2-(4-carboxamido-phenoxy)ethoxy]-5-methoxy-1-(toluene-4-sulphonyl)-1H-indazole, 5-methoxy-1-(toluene-4-sulphonyl)-3-[5-(3-trifluoromethylphenyl)-1,2,4-oxadiazol-3-ylmethoxy]-1H-indazole, 3-(4-benxyloxy-benzyloxy)-5-methoxy-1-(toluene-4-sulphonyl)-1H-indazole, 3-(5-acetyl-1-methoxybenzyloxy)-5-methoxy-1-(toluene-4-sulphonyl)-1H-indazole, 3-(2-chloro-6-fluorobenzyloxy-5-methoxy-1-(toluene-4-sulphonyl)-1H-indazole, 1-(4-chloroben-zenesulphonyl)-3-(2-chloro-6-fluorobenzyloxy)-5-methoxy-1H-indazole, 1-(4-fluorobenzenesulphonyl)-5-methoxy-3-(3-trifluoromethylbenzyloxy)-1H-indazole, 3-(2-fluorobenzyloxy)-5-methoxy-1-toluene-4-sulphonyl)-1H-indazole, 1-(4-fluoro-benzenesulphonyl)-3-(2-fluorobenzyloxy)-5-methoxy-1H-indazole,
1-(4-fluorobenzenesulphonyl)-3-(4-fluorobenzyloxy)-5-methoxy-1H-indazole, 3-(4-chloro-2-nitrobenzyloxy)-5-methoxy-1-(toluene-4-sulphonyl)-1H-indazole, 3-(6-chloro-benzene[1,3]dioxo-5-ylmethoxy)-5-methoxy-1-(toluene-4-sulphonyl)-1H-indazole, 3-(6-fluoro-4H-benzo[1,3]dioxin-8-ylmethoxy)-5-methoxy-1-(toluene-4-sulphonyl)-1H-indazole, 5-methoxy-3-(6-nitro-4H-benzo[1,3]dioxin-8-ylmethoxy)-1-(toluene-4-sulphonyl)-1H-indazole, 5-methoxy-3-(6-nitro-4H-benzo[1,3]dioxin-8-ylmethoxy)-1-(4-fluorobenzenesulphonyl)-1H-indazole, 3-(3,5-dimethyl-isoxazol-4-ylmethoxy)5-methoxy-1-(toluene-4-sulphony)-1H-indazole, 3-(3,5-dimethyl-isoxazol-4-ylmethoxy)-5-methoxy-1-(4-chlorobenzenesulphonyl)-1H-indazole, 5-methoxy-3-[5-(2-methoxyphenyl)[1,2,4]oxadiazol-3-ylmethyl]-1-(toluene-4-sulphonyl)-1H-indazole, 5-methoxy-3-[5, (4-tert-butylphenyl)[1,2,4]oxadiazol-3-ylmethyl]-1-(toluene-4-sulphonyl)-1H-indazole, 3-[2-(4-chlorophenyl)-thiazol-4-ylmethyl]-5-methoxy-1-(toluene-4-sulphony)-1H-indazole, 6-{2-[5-methoxy-1-(toluene-4-sulphonyl)-1H-indazol-3-yloxy]acetyl}-3,4-dihydro-1H-quinolin-2-one, 3-[2-(4-chlorophenyl)-2-oxo-ethoxy]-5-methoxy-1-(toluene-4-sulphonyl)-1H-indazole, 3-[2-(4-chlorophenyl)-2-oxo-ethoxy]-5-methoxy-1-(4-methoxybenzenesulphonyl)-1H-indazole, 3-[2-(4-chlorophenyl)-2-oxo-ethoxy]-5-methoxy-1-(4-chlorobenzenesulphonyl)-1H-indazole, 3-[2-(3,4-dichlorophenyl)-2-oxo-ethoxy]-5-methoxy-1-(toluene-4-sulphonyl)-1H-indazole, 3-[2-(3,4-dichlorophenyl)-2-oxo-ethoxy]-5-methoxy-1-(4-chlorobenzenesulphonyl)-1H-indazole, 3-[2-(3,4-dichlorophenyl)-2-oxo-ethoxy]-5-methoxy-1-(4-acetylamino-benzenesulphonyl)-1H-indazole, 3-[2-(3,4-dichlorophenyl)-2-oxo-ethoxy]-5-methoxy-1-(naphthalene-1-sulphonyl)-1H-indazole, 3-[2-(3,4-dichlorophenyl)-2-oxo-ethoxy]-5-methoxy-1-(quinoline-8-sulphonyl)-1H-indazole, 3-[2-(4-diphenyl)-2-oxo-ethoxy]-5-methoxy-1-(toluene-4-sulphonyl)-1H-indazole, 3-[2-(4-diphenyl)-2-oxo-ethoxy]-5-methoxy-1-(4-chlorobenzenesulphonyl)-1H-indazole, 3-[2-(4-diphenyl)-2-oxo-ethoxy]-5-methoxy-1-(4-methoxybenzenesulphonyl)-1H-indazole, 3-[2-(4-diphenyl)2-oxo-ethoxy]-5-methoxy-1-(4-acetylaminobenzenesulphonyl)-1H-indazole, N-(2,4-difluorophenyl)-2-[5-methoxy-1-(toluene-4-sulphonyl)-1H-indazol-3-yloxy]acetamide, 5-methoxy-3-(3-phenylallyloxy)-1-(toluene-4-sulphonyl)-1H-indazole, 1-(2,4-dichlorophenyl)-2-[5-methoxy-1-(toluene-4-sulphonyl)-1H-indazol-3-yloxy]-ethanol, 1-(4-methoxyphenyl)-4-[5-methoxy-1-(toluene-4-sulphonyl)-1H-indazol-3-yloxyl]-butan-1-one, 1-(4-methoxyphenyl)-4-[1-(4-fluorobenzenesulphonyl)-5-methoxy-1H-indazol-3-yloxy]-butan-1-one, 1-(4-fluorophenyl)-4-[5-methoxy-1-(toluene-4-sulphonyl)-1H-indazol-3-yloxy]-butan-1-one, N-(4-methoxyphenyl)-2-[5-methoxy-1-(toluene-4-sulphonyl)-1H-indazol-3-yloxy]-acetamide, N-(2,4-dichlorophenyl)-2-[5-methoxy-1-(toluene-4-sulphonyl)-1H-indazol-3-yloxy]-acetamide, N-benzyl-2-[5-methoxy-1-(toluene-4-sulphonyl)-1H-indazol-3-yloxy)-acetamide, 3-{2-[5-methoxy-1-(toluene-4-sulphonyl)-1H-indazol-3-yloxy]ethyl}-1H-quinazoline-2,4,dione, 3-{2-[5-methoxy-1-(4-methoxybenzenesulphonyl)-1H-indazol-3-yloxy]ethyl}-1H-quinazoline-2,4-dione, 3-(6-chlorobenzo[1,3]dioxol-5-ylmethoxy)-5-methoxy-1-(3-nitrobenzyl)-1H-indazole, 3-[2-(4-chlorophenoxy)ethoxy]-5-methoxy-1-(3-nitrobenzyl)-1H-indazole, 3-[2-(4-nitrophenoxy)ethoxy]-5-methoxy-1-(3-nitrobenzyl)-1H-indazole, 3-[2-(4-carboxamino-phenoxy)ethoxy]-5-methoxy-1-(3-nitrobenzyl)-1H-indazole, 3-[2-(2-bromo-4,6-difluorophenoxy)ethoxy]-5-methoxy-1-(3-nitrobenzyl)-1H-indazole, 3-[2-(2,6-dibromo-4-nitrophenoxy)ethoxy]-5-methoxy-1-(3-nitrobenzyl)-1H-indazole, 5-methoxy-1-(3-nitrobenzyl)-3-(pyridin-4-ylmethoxy)-1H-indazole hydrochloride, 3-(2,6-dichlorobenzyloxy)-5-methoxy-1-(3-nitrobenzyl)-1H-indazole, 3-[2- (4-chlorophenyl)acetoxy]-5-methoxy-1-(3-nitrobenzyl)-1H-indazole 3-[3-(6-chlorobenzo[1,3]dioxol-5-ylmethoxy)-5-methoxyindazol-1-ylmethyl)-phenylamine, 4-{2-[1-(3-aminobenzyl)-5-methoxy-1H-indazol-3-yloxy]-ethoxy}-phenylamine dihydrochloride, 4-{2-[1-(3-aminobenzyl)-5-methoxy-1H-indazol-3-yloxy]-ethoxy}-benzamide, 3-[3-(4-chlorophenylcarboxymethoxyloxy)-5-methoxy-indazol-1-ylmethyl]-phenylamine hydrochloride, 1-(4-fluorobenzyl)-5-methoxy-3-[2-(4-nitrophenoxy)ethoxy]-1H-indazole, 3-[2-(4-chlorophenoxy)ethoxy]-1-(4-fluorobenzyl)-5-methoxy-1H-indazole, 3-[2-(4-aminophenoxy)ethoxy]-1-(4-fluorobenzyl)-5-methoxy-1H-indazole, 3-[2-(2-bromo-4,6-difluorophenoxy)ethoxy]-1-(4-fluorobenzyl)-5-methoxy-1H-indazole, 3-(3,5-dimethylisoxazol-4-ylmethoxy)-1-(4-fluorobenzyl)-5-methoxy-1H-indazole, 3-(2,3,6-trifluorobenzyloxy)-1-(4-fluorobenzyl)-5-methoxy-1H-indazole, 3-(6-chlorobenzo[1,3]dioxol-5-ylmethoxy)-1-(4-fluorobenzyl)-5-methoxy-1H-indazole, 3-(4-chlorophenylcarboxymethyloxy)-1-(4-fluorobenzyl)-5-methoxy-1H-indazole,3-[2-(2-bromo-4,6-difluorophenoxy)ethoxy]-5-methoxy-1-[2-(4-nitrophenoxy)ethyl]-1H-indazole, 3-[2-(4-chlorophenoxy)ethoxy]-5-methoxy-1-[2-(4-nitrophenoxy)ethyl]-1H-indazole, 3-[2-(2,6-dibromo-4-nitrophenoxy)ethoxy]-5-methoxy-1-[2-(4-nitrophenoxy)ethyl]-1H-indazole, 3-(4-chlorophenylcarboxymethyloxy)-5-methoxy-1-[2-(4-nitrophenoxy)ethyl]-1H-indazole, 3-(3,4-dichlorophenylcarboxymethyloxy)-5-methoxy-1-[2-(4-nitrophenoxy)ethyl]-1H-indazole, 3-(4-diphenylcarboxymethyloxy)-5-methoxy-1-[2-(4-nitrophenoxyl)ethyl]-1H-indazole, 6-(2-{5-methoxy-1-[2-(4-nitrophenoxy)ethyl]-1H-indazol-3-yloxy} acetyl]-3,4-dihydro-1H-quinolin-2-one, N-(2,4-difluorophenyl)-2-{5-methoxy-1-[2-(4-nitrophenoxy)ethyl]-1H-indazol-3-yloxy}acetamide, 3-(3-acetyl-6-methoxybenzyloxy)-5-methoxy-1-[2-(4-nitrophenoxy)ethyl]-1H-indazole, 3-(3-trifluoromethylbenzyloxy)-5-methoxy-1-[2-(4-nitrophenoxy)ethyl]-1H-indazole, 3-(2-fluoromethylbenzyloxy)-5-methoxy-1-[2-(4-nitrophenoxy)ethyl]-1H-indazole, 3-(6-fluoro-4H-benzo[1,3]dioxin-8-ylmethoxy)-5-methoxy-1-[2-(4-nitrophenoxy)ethyl]-1H-indazole, 3-[2-(4-chlorophenoxy)ethoxy]-1-[2-(4-chlorophenoxy)ethyl]-5-methoxy-1H-indazole, 3-(4-chlorophenoxyalkyloxy)-5-amino-1-(toluene-4-sulphonyl)-1H-indazole, 3-(2,6-dibromo-4-nitrophenoxyalkyloxy)-5-amino-1-(toluene-4-sulphonyl)-1H-indazole and 3-(4-chlorophenylcarboxymethyloxy)-5-amino-1-(toluene-4-sulphonyl)-1H-indazole.

**3.** Compounds according to either of the preceding claims, selected from 3-[2-(2-bromo-4,6-difluorophenoxy)ethoxy]-5-methoxy-1-(toluene-4-sulphonyl)-1H-indazole, 5-methoxy-1-(toluene-4-sulphonyl)-3-[5-(3-trifluoromethylphenyl)-1,2,4-oxadiazol-3-ylmethoxy]-1H-indazole, 6-{2-[5-methoxy-1-(toluene-4-sulphonyl)-1H-indazol-3-yloxy]acetyl}-3,4-dihydro-1H-quinolin-2-one, N-(2,4-difluorophenyl)-2-[5-methoxy-1-(toluene-4-sulphonyl)-1H-indazol-3-yloxy]acetamide, 3-(6-chlorobenzo[1,3]dioxol-5-ylmethoxy)-5-methoxy-1-(3-nitrobenzyl)-1H-indazole, 3-[3-(4-fluorophenyl)-propoxy]-5-nitro-1-(3-nitrobenzyl)-1H-indazole, 3-[3-(6-chlorobenzo[1,3]dioxol-5-ylmethoxy)-5-methoxy-indazol-1-ylmethyl]phenylamine, 1-(4-fluorobenzyl)-5-methoxy-3-[2-(4-nitrophenoxy)ethoxy]-1H-indazole, 3-[2-(2-bromo-4,6-difluorophenoxy)ethoxy]-5-methoxy-1-[2-(4-nitrophenoxy]ethyl]-1H-indazole, 3-[2-(2-bromo-4,6-difluorophenoxy)ethoxy]-1-[3,4-dichlorobenzyl]-5-methylthio-1H-indazole, 1-1-{1-(2,4-dichlorobenzyl)-3-[2-(4-nitrophenoxy)ethoxy]-1H-indazol-5-yl}-3-naphthalen-1-ylurea.

**4.** Compound according to one of the preceding claims, wherein the compound is 3-[2-(2-bromo-4,6-di-fluorophenoxy)ethoxy]-5-methoxy-1-(toluene-4-sulphonyl)-1H-indazole.

**5.** Compound according to one of the preceding claims, wherein the compound is 3-[2-(4-chloro-phenoxy)ethoxy]-5-methoxy-1-1(4-fluorobenzene-sulphonyl)-1H-indazole.

**6.** Physiologically tolerable salts of the compounds, according to one of Claims 1 to 5, **characterized by** neutralization of the bases with inorganic or organic acids or by neutralization of the acids with inorganic or organic bases or by quaternization of tertiary amines to give quaternary ammonium salts.

**7.** Use of the compounds according to one of Claims 1 to 5 or their salts according to Claim 6 as therapeutic active compounds for the production of medicaments for the treatment of diseases mediated by PPIase.

**8.** Use of the compounds according to one of Claims 1 to 5 or their salts according to Claim 6 as therapeutic active compounds for the production of medicaments for the treatment of diseases which are connected with the suppression of immunological processes.

**9.** Use of the compounds according to one of Claims 1 to 5 or their salts according to Claim 6 as therapeutic active compounds for the production of medicaments for the treatment of psoriasis.

**10.** Use of the compounds according to one of Claims 1 to 5 or their salts according to Claim 6 as therapeutic active compounds for the production of medicaments for the treatment of bronchial asthma, allergic rhinitis, allergic conjunctivitis, atopic dermatitis, eczema, allergic angiitis, inflammations mediated by eosinophils such as eosinophilic fasciitis, eosinophilic pneumonia and PIE syndrome, autoimmune diseases such as rheumatoid arthritis, rheumatoid spondylitis, lupus erythematosus, multiple sclerosis, glomerulonephritis and uveitis, insulin-dependent diabetes mellitus and sepsis.

**11.** Process for the preparation of a compound according to one of Claims 1 to 6, **characterized in that**

a) for X = $-SO_2-$ the reaction is carried out according to scheme 1 by reacting 1H-indazol-3-yl sulphonates **II**

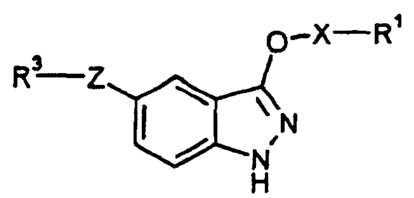

**Formula II**

in the presence of a base and if appropriate in the presence of a diluent to give compounds of the general formula **III**

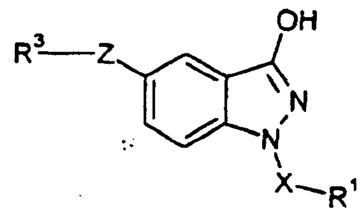

**Formula III**

where $R^1$, $R^3$, X and Z have the meaning mentioned in Claim 1 or above, and reacting 1H-indazol-3-yl sulphonates **II** or 1-sulphonylindazoles **III,** if appropriate in the presence of a base, and if appropriate in the presence of a diluent, with compounds of the general formula Hal-Y-$R^2$, where $R^1$, $R^2$, $R^3$, X, Y and Z have the meaning mentioned in Claim 1 or above and Hal is a halogen atom F, Cl, Br or iodine, to give compounds of the general formula **I** where $R^1$, $R^2$, $R^3$, X, Y and Z have the meaning mentioned in Claim 1 or above,

b) for X = $-(CH_2)_p-$, $-(CH_2)_p-O-$, where p = 1...4 the reaction is carried out according to scheme 2 by reacting compounds of the general formula **III**, if appropriate in the presence of a base, and if appropriate in the presence of a diluent, with compounds of the general formula Hal-Y-$R^2$, where $R^1$, $R^2$, $R^3$, X, Y and Z have the meaning mentioned in Claim 1 or above and Hal is a halogen atom F, Cl, Br or iodine, to give compounds of the general formula **I,** where $R^1$, $R^2$, $R^3$, X, Y and Z have the meaning mentioned in Claim 1 or above,

where **formula III** can also be present as the tautomeric form **formula IV**

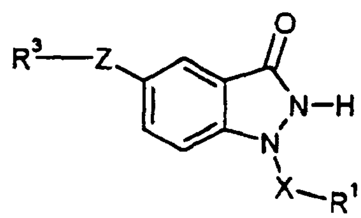

**Formula IV**

according to scheme 3.

**12.** Process according to Claim 11, wherein the base used is sodium hydride and/or the diluent used is dimethyl sulphoxide.

**13.** Pharmaceutical composition, **characterized in that**, as an active constituent, it contains at least one compound according to one of Claims 1 to 6 and physiologically tolerable carriers and/or diluents or auxiliaries.

**14.** Pharmaceutical preparations, **characterized in that**, as an active constituent, they contain at least one compound according to one of Claims 1 to 6 and a suitable carrier.

**15.** Pharmaceutical preparations according to either of Claims 13 or 14, **characterized in that** they are administered in the form of tablets, coated tablets, capsules, aerosols, powder formulations, patches, solutions, ampoules or suppositories.

**16.** Use of a compound according to one of Claims 1 to 6 and/or of pharmaceutical preparations according to either of Claims 13 or 14 for preparing agents having anti-asthmatic, anti-allergic, antiinflammatory and/or immunomodulating actions on their own or in combination with one another or in combination with carriers.

## Revendications

**1.** 1H-indazoles 1,5- et 3-O-substitués de formule générale (I) :

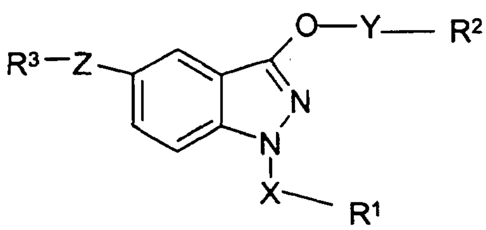

Formule (I)

dans laquelle X, Y, Z, $R^1$, $R^2$ et $R^3$ ont la signification suivante :

X peut être $-SO_2-$, $-(CH_2)_p-$, $-(CH_2)_p-O-$ avec p = 1 à 4,
Y peut être $-(CH_2)_p-$, $-(CH_2)_p-O-$, $-(CH_2)_p-(C=O)-$, $-(CH_2)_p-(C=O)-NH-$, $-(CH_2)_p-CHOH-$, $-(CH_2)_p-CH=CH-$, avec p = 1 à 4,
Z peut être -O-, -S-, -NH-(C=O)-NH-,
$R^1$ peut représenter les groupes suivants :
naphtyle, quinolyle ou phényle, les carbocycles pouvant être substitués par les groupes :
-alkyle en $C_1$-$C_6$, -O-alkyle en $C_1$-$C_6$, -F, -Cl, $-NO_2$, $-NH_2$, -(CO)-alkyle en $C_1$-$C_6$, -S-aryle en $C_6$-$C_{14}$,
$R^1$ peut encore être H, à condition que $R^1$ ne soit pas H, si X = $CH_2$ ;
$R^2$ peut représenter les groupes suivants :
phényle, ou hétérocycle avec 5 à 15 chaînons et 1 à 6 hétéroatomes, mono- ou bicyclique saturé ou insaturé une ou plusieurs fois ;
dans lequel le phényle, respectivement les hétérocycles, peuvent être substitués une ou plusieurs fois avec les groupes :
-alkyle en $C_1$-$C_6$, -O-alkyle en $C_1$-$C_6$, des carbocycles monocycliques saturés ayant 3 à 14 atomes, -F, -Cl, -Br, $-NO_2$, $-NH_2$,-CN, -(CO)-alkyle en $C_1$-$C_6$, benzyloxy, $-CF_3$,
$R^3$ peut représenter les groupes suivants :
méthyle, naphtyle ou phényle, le phényle pouvant être substitué une ou plusieurs fois avec :
-O-alkyle en $C_1$-$C_6$, -Cl.

**2.** 1H-indazoles 1,5- et 3-O-substitués, choisis dans le groupe comprenant les :

3-[2-(2-Bromo-4,6-difluorophénoxy)éthoxy]-5-méthoxy-1-(tolyl-4-sulfonyl)-1H-indazole,
5-méthoxy-3- [2- (phénoxy)éthoxy]-1- (tolyl-4-sulfonyl)-1H-indazole,
5-méthoxy-3-[3-(phénoxy)propoxy]-1-(tolyl-4-sulfonyl)-1H-indazole,
3-[4-(butoxy)propoxy]-5-méthoxy-1-(tolyl-4-sulfonyl)-1H-indazole,
3-[2-(2-bromophénoxy)éthoxy]-5-méthoxy-1-(tolyl-4-sulfonyl)-1H-indazole,
3-[2-(4-chlorophénoxy)éthoxy]-5-méthoxy-1-(tolyl-4-sulfonyl)-1H-indazole,
3-[2-(4-chlorophénoxy)éthoxy]-5-méthoxy-1-(4-chlorobenzènesulfonyl)-1H-indazole,
3-[2-(4-chlorophénoxy)éthoxy]-5-méthoxy-1-(4-fluorobenzènesulfonyl)-1H-indazole,
3-[2-(4-chlorophénoxy)éthoxy]-5-méthoxy-1-(4-méthoxybenzènesulfonyl)-1H-indazole,
3-[2-(4-chlorophénoxy)éthoxy]-5-méthoxy-1-(4-trifluorométhoxybenzènesulfonyl)-1H-indazole,
3-[2-(2,6-dichlorophénoxy)éthoxy]-5-méthoxy-1-(tolyl-4-sulfonyl)-1H-indazole,
3-[2-(2,4-difluorophénoxy)éthoxy]-5-méthoxy-1-(tolyl-4-sulfonyl)-1H-indazole,
3-[2-(2,6-difluorophénoxy)éthoxy]-5-méthoxy-1-(tolyl-4-sulfonyl)-1H-indazole,
5-méthoxy-1-(tolyl-4-sulfonyl)-(3-[2-(2,4,6-trifluorophénoxy)éthoxy]-1H-indazole,
3-[2-(2-bromo-4,6-difluorophénoxy)éthoxy]-5-méthoxy-1-(4-méthoxybenzènesulfonyl)-1H-indazole,
3-[2-(2-bromo-4,6-difluorophénoxy)éthoxy]-5-méthoxy-1-(4-chlorobenzènesulfonyl)-1H-indazole,
3-[2-(2,6-dibromo-4-nitrophénoxy)éthoxy]-5-méthoxy-1-(tolyl-4-sulfonyl)-1H-indazole,
3-[2-(2,6-dibromo-4-nitrophénoxy)éthoxy]-5-méthoxy-1-(4-méthoxybenzènesulfonyl)-1H-indazole,
5-methoxy-3-[2-(4-nitrophénoxy)éthoxy]-1-(tolyl-4-sulfonyl)-1H-indazole,

1-(4-chlorobenzènesulfonyl)-5-méthoxy-3-[2-(4-nitrophénoxy)éthoxy]-1H-indazole,
5-méthoxy-1-(4-méthoxybenzènesulfonyl)-3-[2-(4-nitrophénoxy)éthoxy]-1H-indazole,
5-méthoxy-3-[2-(4-nitrophénoxy)éthoxy]-1-(thiophèn-2-sulfonyl)-1H-indazole,
3-[2-(4-cyanophénoxy)éthoxy]-5-méthoxy-1-(tolyl-4-sulfonyl)-1H-indazole,
3-[2-(4-carboxamidophénoxy)éthoxy]-5-méthoxy-1-(tolyl-4-sulfonyl)-1H-indazole,
5-méthoxy-1-(tolyl-4-sulfonyl)-3-[5-(3-trifluorométhylphényl)-1,2,4-oxadiazol-3-ylméthoxy]-1H-indazole,
3-(4-benzyloxy-benzyloxy)-5-méthoxy-1-(tolyl-4-sulfonyl)-1H-indazole,
3-(5-acétyl-2-méthoxybenzyloxy)-5-méthoxy-1-(tolyl-4-sulfonyl)-1H-indazole,
3-(2-chloro-6-fluorobenzyloxy)-5-méthoxy-1-(tolyl-4-sulfonyl)-1H-indazole,
1-(4-chlorobenzènesulfonyl)-3-(2-chloro-6-fluorobenzyloxy)-5-méthoxy-1H-indazole
1-(4-fluorobenzènesulfonyl)-5-méthoxy-3-(3-trifluorométhylbenzyloxy)-1H-indazole,
3-(2-fluorobenzyloxy)-5-méthoxy-1-(tolyl-4-sulfonyl)-1H-indazole,
1-(4-fluorobenzènesulfonyl)-3-(2-fluorobenzyloxy)-5-méthoxy-1H-indazole,
1-(4-fluorobenzènesulfonyl)-3-(4-fluorobenzyloxy)-5-méthoxy-1H-indazole,
3-(4-chloro-2-nitrobenzyloxy)-5-méthoxy-1-(tolyl-4-sulfonyl)-1H-indazole,
3-(6-chlorobenzo[1,3]dioxo-5-ylméthoxy)-5-méthoxy-1-(tolyl-4-sulfonyl)-1H-indazole,
3-(6-fluoro-4H-benzo[1,3]dioxin-8-ylméthoxy)-5-méthoxy-1-(tolyl-4-sulfonyl)-1H-indazole,
5-méthoxy-3-(6-nitro-4H-benzo[1,3]dioxin-8-ylméthoxy)-1-(tolyl-4-sulfonyl)-1H-indazole,
5-méthoxy-3-(6-nitro-4H-benzo[1,3]dioxin-8-ylméthoxy)-1-(4-fluorobenzènesulfonyl)-1H-indazole,
3-(3,5-diméthyl-isoxazol-4-ylméthoxy)-5-méthoxy-1-(tolyl-4-sulfonyl)-1H-indazole,
3-(3,5-diméthyl-isoxazol-4-ylméthoxy)-5-méthoxy-1-(4-chlorobenzènesulfonyl)-1H-indazole,
5-méthoxy-3-[5-(2-méthoxyphényl)[1,2,4]oxadiazol-3-ylméthyl]-1-(tolyl-4-sulfonyl)-1H-indazole,
5-méthoxy-3-[5-(4-tertbutylphényl)[1,2,4]oxadi-azol-3-ylméthyl]-1-(tolyl-4-sulfonyl)-1H-indazole,
3-[2-(4-chlorophényl)-thiazol-4-ylméthyl]-5-méthoxy-1-(tolyl-4-sulfonyl)-1H-indazole,
6-{2-[5-méthoxy-1-(tolyl-4-sulfonyl)-1H-indazol-3-yloxy]acétyle}-3,4-dihydro-1H-quinolin-2-one,
3-[2-(4-chlorophényl)-2-oxo-éthoxy]-5-méthoxy-1-(tolyl-4-sulfonyl)-1H-indazole,
3-[2-(4-chlorophényl)-2-oxo-éthoxy]-5-méthoxy-1-(4-méthoxybenzènesulfonyl)-1H-indazole,
3-[2-(4-chlorophényl)-2-oxo-éthoxy]-5-méthoxy-1-(4-chlorobenzènesulfonyl)-1H-indazole,
3-[2-(3,4-dichlorophényl)-2-oxo-éthoxy]-5-méthoxy-1-(tolyl-4-sulfonyl)-1H-indazole,
3-[2-(3,4-dichlorophenyl)-2-oxo-éthoxy]-5-méthoxy-1-(4-chlorobenzènesulfonyl)-1H-indazole,
3- [2-(3,4-dichlorophényl)-2-oxo-éthoxy]-5-méthoxy-1-(4-acétylamino-benzènesulfonyl)-1H-indazole,
3-[2-(3,4-dichlorophényl)-2-oxo-éthoxy]-5-méthoxy-1-(naphthalèn-1-sulfonyl)-1H-indazole,
3-[2-(3,4-dichlorophényl)-2-oxo-éthoxy]-5-méthoxy-1-(quinoline-8-sulfonyl)-1H-indazole,
3-[2-(4-diphényl)-2-oxo-éthoxy]-5-méthoxy-1-(tolyl-4-sulfonyl)-1H-indazole,
3-[2-(4-diphényl)-2-oxo-éthoxy]-5-méthoxy-1-(4-chlorobenzènesulfonyl)-1H-indazole,
3-[2-(4-diphényl)-2-oxo-éthoxy]-5-méthoxy-1-(4-méthoxybenzènesulfonyl)-1H-indazole,
3-[2-(4-diphényl)-2-oxo-éthoxy]-5-méthoxy-1-(4-acétylaminobenzènesulfonyl)-1H-indazole,
N-(2,4-difluorophényl)-2-[5-méthoxy-1-(tolyl-4-sulfonyl)-1H-indazol-3-yloxy]-acétamide,
5-méthoxy-3-(3-phénylallyloxy)-1-(tolyl-4-sulfonyl)-1H-indazole,
1-(2,4-dichlorophényl)-2-[5-méthoxy-1-(tolyl-4-sulfonyl)-1H-indazol-3-yloxy]-éthanol,
1-(4-méthoxyphényl)-4-[5-méthoxy-1-(tolyl-4-sulfonyl)-1H-indazol-3-yloxy]-butan-1-one,
1-(4-méthoxyphényl)-4-[1-(4-fluorobenzènesulfo-nyl)-5-méthoxy-1H-indazol-3-yloxy]-butan-1-one,
1-(4-fluorophényl)-4-[5-méthoxy-1-(tolyl-4-sulfonyl)-1H-indazol-3-yloxy]-butan-1-one,
N-(4-méthoxyphényl)-2-[5-méthoxy-1-(tolyl-4-sulfonyl)-1H-indazol-3-yloxy]-acétamide,
N-(2,4-dichlorophényl)-2-[5-méthoxy-1-(tolyl-4-sulfonyl)-1H-indazol-3-yloxy]-acétamide,
N-benzyl-2-[5-méthoxy-1-(tolyl-4-sulfonyl)-1H-indazol-3-yloxy]-acétamide,
3-{2-[5-méthoxy-1-(tolyl-4-sulfonyl)-lH-indazol-3-yloxy]éthyl}-1H-quinazoline-2,4-dione,
3-{2-[5-méthoxy-1-(4-méthoxybenzènesulfonyl)-1H-indazol-3-yloxy]éthyl}-1H-quinazoline-2,4-dione,
3-(6-chlorobenzo[1,3]dioxol-5-ylméthoxy)-5-méthoxy-1-(3-nitrobenzyl)-1H-indazole,
3-[2-(4-chlorophénoxy)éthoxy]-5-méthoxy-1-(3-nitrobenzyl)-1H-indazole,
3-[2-(4-nitrophénoxy)éthoxy]-5-méthoxy-1-(3-nitro-benzyl)-1H-indazole,
3-[2-(4-carboxoaminophénoxy)éthoxy]-5-méthoxy-1-(3-nitrobenzyl)-1H-indazole,
3-[2-(2-bromo-4,6-difluorophénoxy)éthoxy]-5-méth-oxy-1-(3-nitrobenzyl)-1H-indazole,
3-[2-(2,6-dibromo-4-nitrophénoxy)éthoxy]-5-méthoxy-1-(3-nitrobenzyl)-1H-indazole,
hydrochlorure de 5-méthoxy-1-(3-nitrobenzyl)-3-(pyridin-4-ylméthoxy)-1H-indazole,
3-(2,6-dichlorobenzyloxy)-5-méthoxy-1-(3-nitro-benzyl)-1H-indazole,
3-[2-(4-chlorophényl)acétoxy]-5-méthoxy-1-(3-nitrobenzyl)-1H-indazole,
3-[3-(6-chlorobenzo[1,3]dioxol-5-ylméthoxy)-5-méthoxy-indazol-1-ylméthyl]-phénylamine,

dihydrochlorure de 4-{2[1-(3-aminobenzyl)-5-méthoxy-1H-indazol-3-yloxy]-éthoxy}-phénylamine,
4-{2[1-(3-aminobenzyl)-5-méthoxy-1H-indazol-3-yloxy]-éthoxy}-benzamide,
hydrochlorure de 3-[3-(4-chlorophénylcarboxy-méthyloxy)-5-méthoxy-indazol-1-ylméthyl]-phénylamine,
1-(4-fluorobenzyl)-5-méthoxy-3-[2-(4-nitrophén-oxy)éthoxy]-1H-indazole,
3-[2-(4-chlorophénoxy)éthoxy]-1-(4-fluorobenzyl)-5-méthoxy-1H-indazole,
3-[2-(4-aminophénoxy)éthoxy]-1-(4-fluorobenzyl)-5-méthoxy-1H-indazole,
3-[2-(2-bromo-4,6-difluorophénoxy)éthoxy]-1-(4-fluorobenzyl)-5-méthoxy-1H-indazole,
3-(3,5-diméthylisoxasol-4-ylméthoxy)-1-(4-fluorobenzyl)-5-méthoxy-1H-indazole,
3-(2,3,6-trifluorobenzyloxy)-1-(4-fluorobenzyl)-5-méthoxy-1H-indazole,
3-(6-chlorobenzo[1,3]dioxol-5-ylméthoxy)-1-(4-fluorobenzyl)-5-méthoxy-1H-indazole,
3-(4-chlorophénylcarboxyméthyloxy)-1-(4-fluoro-benzyl)-5-méthoxy-1H-indazole,
3-[2-(2-bromo-4,6-difluorophénoxy)éthoxy]-5-méth-oxy-1-[2-(4-nitrophénoxy)éthyl]-1H-indazole,
3-[2-(4-chlorophénoxy)éthoxy]-5-méthoxy-1-[2-(4-nitrophénoxy)éthyl]-1H-indazole,
3-[2-(2,6-dibromo-4-nitrophénoxy)éthoxy]-5-méth-oxy-1-[2-(4-nitrophénoxy)éthyl]-1H-indazole,
3-(4-chlorophénylcarboxyméthyloxy)-5-méthoxy-1-[2-(4-nitrophénoxy)éthyl]-1H-indazole,
3-(3,4-dichlorophénylcarboxyméthyloxy)-5-méthoxy-1-[2-(4-nitrophénoxy)éthyl]-1H-indazole,
3-(4-diphénylcarboxyméthyloxy)-5-méthoxy-1-[2-(4-nitrophénoxy)éthyl]-1H-indazole,
6-(2-{5-méthoxy-1-[2-(4-nitrophénoxy)éthyl]-1H-indazol-3-yloxy}acétyl)-3,4-dihydro-1H-quinolin-2-one,
N-(2,4-difluorophényl)-2-{5-méthoxy-1-[2-(4-nitrophénoxy)éthyl]-1H-indazol-3-yloxy}-acétamide,
3-(3-acétyl-6-méthoxybenzyloxy)-5-méthoxy-1-[2-(4-nitrophénoxy)éthyl]-1H-indazole,
3-(3-trifluorométhylbenzyloxy)-5-méthoxy-1-[2-(4-nitrophénoxy)éthyl]-1H-indazole,
3-(2-fluorométhylbenzyloxy)-5-méthoxy-1-[2-(4-nitrophénoxy)éthyl]-1H-indazole,
3-(6-fluoro-4H-benzo[1,3]dioxin-8-ylméthoxy)-5-méthoxy-1-[2-(4-nitrophénoxy)éthyl]-1H-indazole,
3-[2-(4-chlorophénoxy)éthoxy]-1-[2-(4-chloro-phénoxy)éthyl]-5-méthoxy-1H-indazole,
3-(4-chlorophénoxyalkyloxy)-5-amino-1-(tolyl-4-sulfonyl)-1H-indazole,
3-(2,6-dibromo-4-nitrophénoxyalkyloxy)-5-amino-1-(tolyl-4-sulfonyl)-1H-indazole et
3-(4-chlorophénylcarboxyméthyloxy)-5-amino-1-(tol-yl-4-sulfonyl)-1H-indazole.

**3.** Composés selon l'une quelconque des revendications précédentes, choisis dans le groupe composé de :

3-[2-(2-bromo-4,6-difluorophénoxy)éthoxy]-5-méth-oxy-1-(tolyl-4-sulfonyl)-1H-indazole,
5-méthoxy-1-(tolyl-4-sulfonyl)-3-[5-(3-trifluoro-méthylphényl)-1,2,4-oxadiazol-3-ylméthoxy]-1H-indazole,
6-{2-[5-méthoxy-1-(tolyl-4-sulfonyl)-1H-indazol-3-yloxy]acétyl}-3,4-dihydro-1H-quinolin-2-one,
N-(2,4-difluorophényl)-2-[5-méthoxy-1-(tolyl-4-sulfonyl)-1H-indazol-3-yloxy]-acétamide,
3-(6-chlorobenzo[1,3]dioxol-5-ylméthoxy)-5-méth-oxy-1-(3-nitrobenzyl)-1H-indazole,
3-[3-(4-fluorophényl)-propoxy]-5-nitro-1-(3-nitro-benzyl)-1H-indazole,
3-[3-(6-chlorobenzo[1,3]dioxol-5-ylméthoxy)-5-méthoxy-indazol-1-ylméthyl]-phénylamine,
1-(4-fluorobenzyl)-5-méthoxy-3-[2-(4-nitrophén-oxy)éthoxy]-1H-indazole,
3-[2-(2-Bromo-4,6-difluorophénoxy)éthoxy]-5-méth-oxy-1-[2-(4-nitrophénoxy)éthyl]-1H-indazole,
3-[2-(2-Bromo-4,6-difluorophénoxy)éthoxy]-1-[3,4-dichlorobenzyl]-5-méthylthio-1H-indazole et
1-1-{1-(2,4-dichlorobenzyl)-3-[2-(4-nitrophénoxy)éthoxy]-1H-indazol-5-yl}-3-naphthalèn-1-ylurée,

**4.** Composé selon l'une quelconque des revendications précédentes, lequel composé est le 3-[2-(2-bromo-4,6-difluorophénoxy)éthoxy]-5-méthoxy-1-(tolyl-4-sulfonyl)-1H-indazole.

**5.** Composé selon l'une quelconque des revendications précédentes, lequel composé est le 3-[2-(4-chlorophénoxy) éthoxy]-5-méthoxy-1-(4-fluorobenzènesulfonyl)-1H-indazole.

**6.** Sels physiologiquement compatibles des composés selon l'une quelconque des revendications 1 à 5, **caractérisés en ce qu'**ils sont obtenus par neutralisation des bases avec des acides inorganiques ou organiques, respectivement par neutralisation des acides avec des bases inorganiques ou organiques, respectivement par quaternisation des amines tertiaires en sels d'ammonium quaternaires.

**7.** Utilisation des composés selon l'une quelconque des revendications 1 à 5 ou de leurs sels selon la revendication 6 en tant que principes actifs à usage thérapeutique pour la fabrication de médicaments destinés au traitement des maladies médiées par la PPIase.

**8.** Utilisation des composés selon l'une quelconque des revendications 1 à 5 ou de leurs sels selon la revendication

6 en tant que principes actifs à usage thérapeutique pour la fabrication de médicaments destinés au traitement des maladies qui sont liées à l'inhibition des processus immunitaires.

**9.** Utilisation des composés selon l'une quelconque des revendications 1 à 5 ou de leurs sels selon la revendication 6 en tant que principes actifs à usage thérapeutique pour la fabrication de médicaments destinés au traitement du psoriasis.

**10.** Utilisation des composés selon l'une quelconque des revendications 1 à 5 ou de leurs sels selon la revendication 6 en tant que principes actifs à usage thérapeutique pour la fabrication de médicaments destinés au traitement de l'asthme bronchique, des rhinites allergiques, de la conjonctivite allergique, de la dermatite atopique, des eczémas, de l'angéite allergique, des inflammations médiées par les éosinophiles comme la fasciite la fasciite à éosinophiles, de la pneumonie à éosinophiles et du syndrome de PIE, des maladies autoimmunes comme l'arthrite rhumatoïde, de la spondylarthrite ankylosante, du lupus érythémateux, la sclérose en plaques, de la glomérulonéphrite et de l'uvéite, du diabète sucré insulinodépendant et de la sepsie.

**11.** Procédé de préparation d'un composé selon l'une quelconque des revendications 1 à 6, **caractérisé en ce que**

a) pour X= $-SO_2-$ le schéma 1 correspondant sera utilisé, dans lequel l'acide sulfonique-1H-indazol-3-ylester (II)

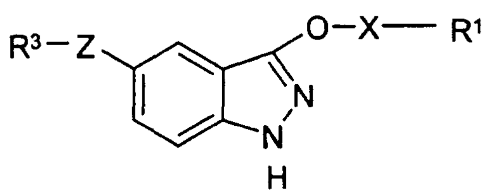

Formule (II)

donnera par réaction, en présence d'une base et le cas échéant en présence d'un diluant, des composés de formule générale (III),

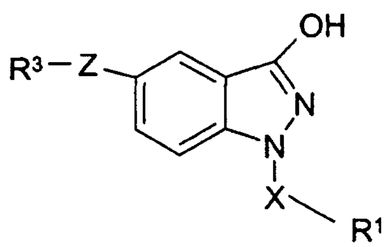

Formule (III)

dans lesquelles $R^1$, $R^3$, X et Z ont la signification indiquée dans la revendication 1 ou ci-dessus, et l'acide sulfonique-1H-indazol-3-ylester (II) ou le 1-sulfonyl-indazole (III) le cas échéant en présence d'une base, et le cas échéant en présence d'un diluant, réagiront avec des composés de formule générale Hal-Y-$R^2$, dans laquelle $R^1$, $R^2$, $R^3$, X, Y et Z ont la signification indiquée dans la revendication 1 ou ci-dessus, et Hal représente un atome halogène de fluor, de chlore, de brome ou d'iode pour donner des composés de formule générale (I) dans laquelle $R^1$, $R^2$, $R^3$, X, Y et Z ont la signification indiquée dans la revendication 1 ou ci-dessus,
b) pour X = $-(CH_2)_p-$, $-(CH_2)_p-O-$ avec p = 1 à 4 le schéma 2 correspondant sera utilisé, dans lequel :

les composés de formule générale (III) le cas échéant en présence d'une base, et le cas échéant en présence d'un diluant, réagiront avec des composés de formule générale Hal-Y-$R^2$, dans laquelle $R^1$, $R^2$, $R^3$, X, Y et Z ont la signification indiquée dans la revendication 1 ou ci-dessus, et Hal représente un atome d'halogène comme le fluor, le chlore, le brome ou l'iode pour donner des composés de formule générale (I) dans laquelle $R^1$, $R^2$, $R^3$, X, Y et Z ont la signification indiquée dans la revendication 1 ou ci-dessus,

dans laquelle la formule (III) peut aussi exister sous forme tautomère de formule (IV)

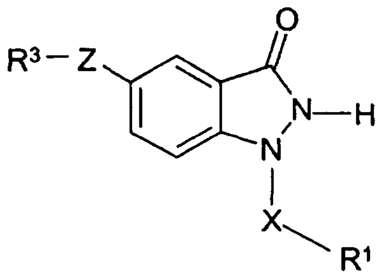

Formule (IV)

correspondant au schéma 3.

**12.** Procédé selon la revendication 11, dans lequel de l'hydrure de sodium est utilisé en tant que base ou/et du diméthylsulfoxyde en tant que diluant.

**13.** Produit pharmaceutique, **caractérisé en ce qu'**il contient comme principe actif au moins un composé selon l'une quelconque des revendications 1 à 6 et des excipients et/ou diluants respectivement adjuvants physiologiquement acceptables.

**14.** Préparations pharmaceutiques **caractérisées en ce qu'**elles contiennent comme principe actif au moins un composé selon l'une quelconque des revendications 1 à 6 et un excipient approprié.

**15.** Préparations pharmaceutiques d'après l'une des revendications 13 ou 14, **caractérisées en ce qu'**elles sont administrées sous forme de comprimés, dragées, gélules, aérosols, formulations en poudre, pansements, solutions, ampoules ou suppositoires.

**16.** Utilisation d'un composé selon l'une quelconque des revendications 1 à 6 et/ou de préparations pharmaceutiques selon l'une des revendications 13 ou 14 pour la préparation d'un produit avec des actions antiasthmatiques, antiallergiques, anti-inflammatoires et/ou d'immunomodulation, seul(es) ou combiné(es) avec d'autres ou combiné (es) avec des excipients.